# FASCICULE DE BREVET EUROPEEN

(11) **EP 3 368 055 B1**
(45) Date de publication et mention de la délivrance du brevet: **18.11.2026**
(21) Numéro de dépôt: 16794958.5
(22) Date de dépôt: 27.10.2016
(51) Int. Cl.: A61K 36/87, A61K 31/197, A61K 31/353, A61K 36/45, A61P 25/24, A61P 25/28, A61P 25/18, A61P 3/10, A61P 25/16, A23L 33/105, A61K 9/20, A61K 9/48, A61K 31/05, A61K 31/192, A61K 31/7048, A61K 45/06

(54) **AGENT NUTRITIONNEL OU THERAPEUTIQUE PARTICULIER COMPRENANT UN MELANGE DE RAISIN ET DE BLEUET**
SPEZIFISCHE NÄHRSTOFF- ODER THERAPEUTISCHE MITTEL MIT EINER MISCHUNG AUS TRAUBEN UND SCHWARZBEERE
SPECIFIC NUTRITIONAL OR THERAPEUTIC AGENT INCLUDING A MIXTURE OF GRAPE AND BLUEBERRY

(30) Priorité: 27.10.2015 FR 1560263
(43) Date de publication de la demande: 05.09.2018
(73) Titulaire: Activ'Inside, 33750 Beychac-et-Caillau (FR); Spécialités Pet Food, 56250 Elven (FR); Université Laval, Québec, QC G1V 0A6 (CA); Institut National De La Recherche Scientifique (INRS), Québec, QC G1K 9A9 (CA)
(72) Inventeur: GAUDOUT, David, 33360 Carignan de Bordeaux (FR); REY, Stéphane, 26200 Montélimar (FR); LEMAIRE, Benoit, 33500 Libourne (FR); BENSALEM, Julien, 33150 Cenon (FR); LEPOUDERE, Anne, 56380 Guer (FR); LETHUILLIER, Delphine, 56400 Plougoumelen (FR); PARADIS, Marie-Eve, Quebec G1K 2V6 (CA); DUDONNE, Stéphanie, Quebec G2C 0L2 (CA); DESJARDINS, Yves, Quebec G1W 3G4 (CA); CALON, Frédéric, Quebec G1S 3Y6 (CA); DAL-PAN, Alexandre, Quebec G1K 3V2 (CA); RAMASSAMY, Charles, Quebec H7V 1B7 (CA)
(74) Mandataire: Aquinov
(86) Numéro de dépôt international: PCT/EP2016/075905
(87) Numéro de publication internationale: WO 2017/072219

(56) Documents cités:
- WO-A1-2006/083666
- US-A1- 2006 024 385
- US-A1- 2008 213 401
- JULIEN BENSALEM ET AL: "Abstract", JOURNAL OF NUTRITIONAL SCIENCE, vol. 7, 1 January 2018 (2018-01-01), XP055605509, DOI: 10.1017/jns.2018.10
- ANONYMOUS: "Optimized Curcumin with Neurophenol TM- 60 Vegetarian capsules", 14 May 2015 (2015-05-14), pages 2PP, XP002757330, Retrieved from the Internet <URL:https://www.pureformulas.com/optimized-curcumin-with-neurophenol-60-vegetarian-capsules-by-douglas-laboratories.html> [retrieved on 20160509]
- DOUGLAS LABORATORIES: "Optimized Curcumin with Neurophenol(TM)", 1 January 2015 (2015-01-01), XP055270332, Retrieved from the Internet <URL:http://www.douglaslabs.com/media/DL202028.pdf> [retrieved on 20160503]
- DATABASE WPI Week 201316, Derwent World Patents Index; AN 2013-B84395, XP002757331
- MARTINEAU ANNE-SOPHIE ET AL: "The consumption of a grape and blueberry polyphenol-rich mixture is safe in dogs", FASEB JOURNAL, vol. 29, no. Suppl. 1, 136.1, April 2015 (2015-04-01), & EXPERIMENTAL BIOLOGY MEETING 2015; BOSTON, MA, USA; MARCH 28 -APRIL 01, 2015, XP002757332
- CHERNIACK E PAUL ED - ROBERFROID MARCEL ET AL: "A berry thought-provoking idea: the potential role of plant polyphenols in the treatment of age-related cognitive disorders", THE BRITISH JOURNAL OF NUTRITION, vol. 108, no. 5, 1 September 2012 (2012-09-01), pages 794 - 800, XP009171713, ISSN: 1475-2662, [retrieved on 20120405], DOI: 10.1017/S0007114512000669

## Description

La présente invention concerne un mélange de polyphénols spécifiques présentant une action synergique notamment sur les fonctions cognitives et les fonctions exécutives chez l'Homme et l'animal.

L'invention vise également l'utilisation de ce mélange pour améliorer les fonctions cognitives et les fonctions exécutives, retarder le déclin cognitif et prévenir et lutter contre les pathologies associées au déclin cognitif chez l'Homme et l'animal.

On sait que la vieillesse est associée à des troubles cognitifs et à des déficiences neurodégénératives telles que la maladie d'Alzheimer et la maladie de Parkinson. Comme la population âgée augmente, il y a une prévalence de ces troubles liés à l'âge. Il est donc important de développer des solutions pour prévenir ou minimiser le déclin cognitif lié à l'âge et retarder l'apparition des pathologies associées.

À cette fin, une prévention basée sur la nutrition a été suggérée ces dernières années pour éviter ou retarder l'évolution vers la démence et donc maintenir un état cognitif stable et un bien-être chez les personnes âgées. La recherche dédiée à la compréhension de la relation entre la nutrition et le « vieillissement en bonne santé » s'est ainsi intensifiée et parmi les aliments étudiés, les fruits et légumes riches en polyphénols ont été identifiés comme capables de retarder les déficits physiologiques et fonctionnels liés à l'âge et protéger l'Homme ou l'animal contre les maladies dégénératives associées (tel que décrit par exemple dans : JA Joseph, Shukitt-Hale B , G. Casadesus « Inverser les effets délétères du vieillissement sur la communication et le comportement neuronal: propriétés bénéfiques de composés polyphénoliques des fruits » de l'American Journal of Clinical Nutrition 2005; 81 (Suppl 1): 3135-65.). En effet, les polyphénols, en particulier les flavonoïdes, sont connus pour leur capacité à améliorer les processus d'apprentissage et de mémoire et sont aujourd'hui largement étudiés pour leur potentiel dans la prévention du déclin cognitif lié à l'âge aussi bien chez les animaux que chez l'Homme. Bien que les mécanismes d'action des flavonoïdes ne soient pas clairement identifiés, on sait qu'ils sont capables de moduler les processus cellulaires et moléculaires impliqués dans l'apprentissage et la mémoire.

Parmi les fruits contenant des polyphénols étudiés pour leur effet sur le décin cognitif lié à l'âge, on peut citer notamment les baies et en particulier le bleuet, les fraises et les raisins. Parmi les différents polyphénols présents dans les baies, ceux qui ont été particulièrement étudiés pour leur effet sur les fonctions cérébrales sont le resvératrol et les flavonoïdes, en particulier les flavanols et les anthocyanes.

Le bleuet est connu pour contenir une grande quantité de polyphénols et possède une capacité antioxydante plus importante que les autres fruits et légumes. De nombreuses études suggèrent que la consommation de bleuet retarde des déficits fonctionnels et physiologiques liés à l'âge. Par exemple, la consommation journalière de jus de bleuet pendant 12 semaines augmente les performances de la mémoire épisodique chez les personnes âgées (Krikorian R, Shidler MD, Nash TA, Kalt W, Vinqvist-Tymchuk MR, Shukitt-Hale B, et al. « Blueberry supplementation improves memory in older adults". Journal of agricultural and food chemistry. 2010;58(7):3996-4000 ).

La fraise possède un fort pouvoir antioxidant et anti-inflammatoire capable de prévenir des altérations neurochimiques et comportementales liées à l'âge.

Le raisin quant à lui est particulièrement riche en flavonoïdes (catéchines, épicatéchines, oligomères proanthocyanidiques, polymères procyanidiques, et anthocyanes) connus pour leurs puissantes capacités antioxydantes. Les propriétés nutritionnelles des raisins comme la source de polyphénols du vin font donc également l'objet de nombreuses études. Similaires aux résultats obtenus avec le jus de bleuet, la consommation de jus de raisin pendant 12 semaines conduit à une amélioration des performances de la mémoire chez les être humains âgés (Krikorian R, Nash TA, Shidler MD, Shukitt-Hale B, Joseph JA. « Concord grape juice supplementation improves memory function in older adults with mild cognitive impairment." The British journal of nutrition. 2010;103(5):730-4).

De plus, des extraits spécifiques de raisin (des extraits de pépins de raisin, par exemple) sont maintenant utilisés comme complément nutritionnel pour leur forte concentration en polyphénols en particulier en flavanols, anthocyanes et resvératrol. Ces extraits sont adaptés pour la supplémentation nutritionnelle, car ils contiennent une concentration plus élevée en polyphénols que les fruits ou les jus, ce qui facilite l'identification de leurs effets et l'étude des mécanismes neurobiologiques sous-jacents.

L'utilisation de baies, de jus ou d'extraits de baies pour retarder le déclin cognitif lié à l'âge et améliorer les fonctions cérébrales est donc connue. Toutefois, lorsque des personnes ou des animaux consomment des baies, des jus ou des extraits de baies existants, la biodisponibilité des polyphénols contenus dans ces produits n'est pas satisfaisante et l'effet sur les fonctions cognitives et fonctionnelles n'est pas suffisant.

Dans ce contexte, il est connu des produits à base d'un mélange de raisin et de myrtille ou des extraits de baies respectivement dans les documents US 2008/213401, WO 2006/083666 et MX 2011 006 005. Le document US 2006/024385 décrit une liste d'antioxydants dont les proanthocyanidines. Il est également connu un produit dénommé Neurophénol^{®} basé sur un mélange de raisin et de bleuet et dépourvu de resvératrol commercialisé par Douglas Laboratories ("Optimized Curcumin with Neurophenol TM- 60 Vegetarian capsules", https://www.pureformulas.com/product/optimized-curcumin-with-neurophenol-by-douglas-laboratories/1000038109 ; "Optimized Curcumin with Neurophenol(TM)", https://www.douglaslabs.com/media/DL202028.pdf). Enfin, l'article Martineau Anne-Sophie et al., "The consumption of a grape and blueberry polyphenol-rich mixture is safe in dogs" avril 2015 décrit la toxicité du bleuet et du raisin chez le chien.Aussi, l'objectif de l'invention est de pallier ces inconvénients en proposant un produit contenant des polyphénols présentant une biodisponibilité améliorée et une efficacité plus importante pour lutter contre le déclin cognitif.

A cet effet, l'invention vise un agent nutritionnel ou thérapeutique comprenant un mélange de molécules obtenu à partir d'un extrait de *Vitis vinifera* et d'un extrait de *Vaccinium angustifolium,* comprenant :
- au moins 1% de catéchine et/ou epicatéchine, le pourcentage étant donné en poids par rapport au poids total du mélange, préférentiellement au moins 5%,
- au moins 5 ppm (parties par million dans le mélange) d'acide férulique, préférentiellement au moins 10 ppm,
- au moins 200ppm de resvératrol,
- au moins 300ppm de la malvidine 3 glucoside,
- au moins 50ppm de quercétine et/ou glycosides de quercétine, et
- au moins 500ppm d'anthocyanidines.

L'agent selon l'invention contient des flavanols, en particulier des catéchine et/ou épicatéchine, connus pour leur effet sur les performances cognitives, comme cela est décrit notamment dans Endeiro C, Vauzour D, Rattray M, Waffo-Teguo P, Merillon JM, Butler LT, et al. « Dietary levels of pure flavonoids improve spatial memory performance and increase hippocampal brain-derived neurotrophic factor" PloS one. 2013;8(5):e63535, ainsi que dans Van Praag H, Lucero MJ, Yeo GW, Stecker K, Heivand N, Zhao C, et al. "Plant-derived flavanol (-)epicatechin enhances angiogenesis and retention of spatial memory in mice" The Journal of neuroscience : the official journal of the Society for Neuroscience. 2007;27(22):5869-78.

De même l'acide férulique est connu pour ses effets sur le système neuronal, en particulier pour protéger les cellules neuronales de la mort cellulaire induite par l'ischémie cérébrale comme cela est décrit notamment dans Cheng CYS, S.Y.; Tang, N.Y.; Ho, T.Y.; Chiang, S.Y.; Hsieh, C.L. . « Ferulic acid provides neuroprotection against oxidative stress-related apoptosis after cerebral ischemia/reperfusion injury by inhibiting ICAM-1 mRNA expression in rats. Brain Res." 2008;1209:136-50. De plus, son activité antioxydante a été testée dans la maladie d'Alzheimer (Sgarbossa A, Giacomazza D, Di Carlo M. « Ferulic Acid: A Hope for Alzheimer's Disease Therapy from Plants. Nutrients ». 2015;7(7):5764-82) et son administration à long terme semble protéger contre les déficits de mémoire et d'apprentissage (Yan JJC, J.Y.; Kim, H.S.; Kim, K.L.; Jung, J.S.; Huh, S.O.; Suh, H.W.; Kim, Y.H.; Song, D.K. « Protection against β-amyloid peptide toxicity in vivo with long-term administration of ferulic acid ». Br J Pharmacol. 2001;133:89-96.)

Or, cet effet est encore augmenté lorsque l'agent selon l'invention comprend également :
- au moins 200 ppm de resvératrol, et
- au moins 50 ppm de quercétine et/ou glycosides de quercétine, et
- au moins 500 ppm d'anthocyanidines.

Ces molécules sont elles aussi connues pour leur effet sur les fonctions cognitives.

Le resvératrol possède lui aussi de nombreuses activités bénéfiques pour l'Homme ou l'animal parmi lesquelles une amélioration de la mémoire de travail, de l'apprentissage et de la mémoire spatiale, l'activité motrice spontanée (Abraham J, Johnson RW. « Consuming a diet supplemented with resveratrol reduced infection-related neuroinflammation and deficits in working memory in aged mice". Rejuvenation research. 2009;12(6):445-53 ; Dal-Pan A, Pifferi F, Marchal J, Picq JL, Aujard F. "Cognitive performances are selectively enhanced during chronic caloric restriction or resveratrol supplementation in a primate". PloS one. 2011;6(1):e16581.). Le résveratrol est particulièrement connu pour être éventuellement présent dans le raisin, mais il n'est pas présent dans tous les extraits de raisins. En effet, le resvératrol est une phytoalexine qui se développe essentiellement sur la peau du raisin de façon très variable et sa présence dans les extraits, comme pour les autres molécules, est également dépendante du procédé d'extraction mis en œuvre.

La quercétine possède aussi une activité neuroprotectrice importante (Dajas F, Andres AC, Florencia A, Carolina E, Felicia RM. « Neuroprotective actions of flavones and flavonols: mechanisms and relationship to flavonoid structural features. Central nervous system agents in medicinal chemistry. » 2013;13(1):30-5.) et on sait également que la consommation d'aliments riches en anthocyanidines permet de prévenir les déficiences de mémoire et d'améliorer les performances cognitives (Barros D, Amaral OB, Izquierdo I, Geracitano L, do Carmo Bassols Raseira M, Henriques AT, et al. « Behavioral and genoprotective effects of Vaccinium berries intake in mice". Pharmacology, biochemistry, and behavior. 2006;84(2):229-34 ; Cho J, Kang JS, Long PH, Jing J, Back Y, Chung KS. "Antioxidant and memory enhancing effects of purple sweet potato anthocyanin and cordyceps mushroom extract. Archives of pharmacal research". 2003;26(10):821-5 ; Ramirez MR, Izquierdo I, do Carmo Bassols Raseira M, Zuanazzi JA, Barros D, Henriques AT. "Effect of lyophilised Vaccinium berries on memory, anxiety and locomotion in adult rats. Pharmacological research : the official journal of the Italian Pharmacological Society". 2005;52(6):457-62.)

De manière surprenante, la combinaison et la quantité spécifique des polyphénols présents dans l'agent selon l'invention présentent un effet synergique et augmentent la biodisponibilité des polyphénols lorsqu'ils sont administrés à l'Homme ou l'animal en comparaison à la biodisponibilité de ces mêmes polyphénols lorsqu'ils sont administrés isolément ou via les extraits de baies existants ou lors de la consommation de raisin ou de bleuet ou à des concentrations différentes dans des produits existants. Les molécules du mélange et par conséquent l'agent selon l'invention présentent en particulier un effet synergique antioxydant et/ou sur l'amélioration des fonctions cognitives et/ou exécutives chez l'Homme ou l'animal.

L'agent selon l'invention est donc particulièrement utile notamment comme médicament chez l'Homme ou l'animal, et peut prévenir et/ou lutter contre des pathologies associées au déclin cognitif.

De même, l'invention concerne aussi l'utilisation d'un tel agent pour des applications nutritionnelles non thérapeutiques chez l'Homme ou l'animal sain, en particulier pour améliorer les fonctions cognitives et/ou exécutives.

L'invention est maintenant décrite en détail en regard des figures annexées sur lesquelles :
- La Figure 1 représente les différences de biodisponibilité de polyphénols dans le plasma de souris, entre une administration aigüe et une administration chronique d'un extrait de *Vitis vinifera,* d'un extrait de *Vaccinium angustifolium* ou d'un agent selon l'invention ;
- La Figure 2 représente la classification ascendante hiérarchique (CAH) des métabolites phénoliques retrouvés dans le plasma de souris avant et après traitement par ingestion chronique d'un extrait de *Vitis vinifera,* d'un extrait de *Vaccinium angustifolium* ou d'un agent selon l'invention ;
- La Figure 3 représente la classification ascendante hiérarchique (CAH) des métabolites phénoliques retrouvés dans les excréments de souris avant et après traitement par ingestion chronique d'un extrait de *Vitis vinifera,* d'un extrait de *Vaccinium angustifolium* ou d'un agent selon l'invention ;
- La Figure 4A représente les effets de l'acide férulique seul sur la protection des cellules neuronales après un traitement aigu ;
- La Figure 4B représente les effets de la catéchine seule sur la protection des cellules neuronales après un traitement aigu ;
- La Figure 4C représente les effets de l'épicatéchine seule sur la protection des cellules neuronales après un traitement aigu ;
- La Figure 4D représente les effets de l'agent selon l'invention sur la protection des cellules neuronales après un traitement aigu ;
- La Figure 5A représente les effets de l'acide férulique seul, de la catéchine seule et de l'épicatéchine seule, sur la protection des cellules neuronales après trois traitements cumulatifs (survie cellulaire) ;
- Les Figures 5B, 5C, 5D, 5E représentent les effets de l'agent selon l'invention sur la protection des cellules neuronales après trois traitements cumulatifs à différentes concentrations (survie cellulaire) ;
- La Figure 5F représente les effets de l'agent selon l'invention sur la protection des cellules neuronales après trois traitements cumulatifs (production de ROS) ;
- La Figure 6 représente le statut antioxidant total (TAS) de chiens adultes ayant été traités avec l'agent selon l'invention, un extrait de de *Vitis vinifera,* un extrait de *Vaccinium angustifolium,* et un contrôle ;
- La Figure 7 représente l'effet sur les fonctions cognitives de chiens d'un agent selon l'invention à différentes doses, en comparaison à un placebo.

L'invention a donc pour objet un agent nutritionnel ou thérapeutique comprenant au moins un mélange de molécules obtenu à partir de *Vitis vinifera* et de *Vaccinium angustifolium,* ledit mélange comprenant :
- au moins 1% de catéchine et/ou epicatéchine, le pourcentage étant donné en poids par rapport au poids total du mélange, préférentiellement au moins 5%, encore plus préférentiellement entre 5% et 50%, notamment entre 7% et 35%,
- au moins 5 ppm (parties par million dans le mélange) d'acide férulique, préférentiellement au moins 10 ppm, encore plus préférentiellement entre 5 ppm et 300 ppm, notamment entre 10 ppm et 100 ppm.

En plus du au moins 1% de catéchine et/ou épicatéchine, et des au moins 5ppm d'acide férulique, le mélange de molécules selon l'invention comprend également :
- au moins 200 ppm de resvératrol, préférentiellement au moins 300 ppm, encore plus préférentiellement au moins 400 ppm, encore plus préférentiellement entre 300 ppm et 6000 ppm, notamment entre 400 et 6000 ppm, et/ou
- au moins 50 ppm de quercétine et/ou glycosides de quercétine, préférentiellement au moins 70 ppm de quercétine et/ou glycoside, notamment entre 50 ppm et 10000 ppm, et/ou
- au moins 500 ppm d'anthocyanidines, préférentiellement au moins 600 ppm, encore plus préférentiellement au moins 700 ppm, encore plus préférentiellement entre 600 ppm et 5000 ppm.

Enfin, la malvidine 3 glucoside est l'anthocyanidine prépondérante. Elle est présente dans l'agent selon l'invention à une concentration d'au moins 300ppm dans le mélange.

Par « agent nutritionnel » au sens de l'invention on entend un ingrédient alimentaire à vocation nutrionnelle utilisé seul ou associé à d'autres ingrédients ou additifs alimentaires dans des formules alimentaires y compris les compléments alimentaires destinés à l'Homme ou à l'animal.

Par « agent thérapeutique » au sens de l'invention on entend un actif employé à des fins thérapeutiques utilisé seul ou associé à d'autres substances actives ou non dans des formules médicamenteuses y compris de phytothérapie, destiné à l'Homme ou à l'animal.

Par « anthocyanidine » au sens de l'invention on entend toutes les anthocyanes ou anthocyanines ou anthocanthocyanosides, de forme aglycone ou glycosylée (c'est-à-dire portant des sucres). Ainsi, dans la présente demande et au sens de la présente invention, les termes « anthocyanidine », « anthocyane », « anthocyanines » et « anthocanthocyanosides » sont équivalents.

Par « au moins X% de catéchines et/ou épicatéchines » on entend soit au moins X% de catéchines s'il n'y a pas d'épicatéchines dans le mélange, soit au moins X% d'épicatéchines s'il n'y a pas de catéchines dans le mélange, soit au moins X% du mélange de catéchines et d'épicatéchines si à la fois des catéchines et des épicatéchines sont présentes dans le mélange. Préférentiellement, on entend au moins X% du mélange de catéchines et d'épicatéchines.

Par « ppm » on entend parties par million (mg/kg) dans le mélange. Sauf indication contraire, ppm se réfère à un poids par rapport au poids total du mélange.

Selon un premier mode de réalisation, le mélange de molécules est un mélange constitué par un extrait de *Vitis vinifera* et un extrait de *Vaccinium angustifolium.*

Selon un deuxième mode de réalisation (hors invention), le mélange de molécules est un mélange constitué par un extrait obtenu à partir d'un mélange de *Vitis vinifera* et de *Vaccinium angustifolium.*

Selon un troisième mode de réalisation (hors invention), le mélange de molécules est un mélange constitué par :
- un extrait de *Vitis vinifera* et/ou un extrait de *Vaccinium angustifolium,* et
- un extrait obtenu à partir d'un mélange de *Vitis vinifera* et de *Vaccinium angustifolium.*

Par « extrait de *Vitis vinifera »* au sens de l'invention on entend au moins une molécule, préférentiellement un ensemble de molécules, obtenue(s) à partir de *Vitis vinifera.* La matière première peut-être les feuilles et/ou les fruits et/ou les pépins et/ou les bois, préférentiellement la matière première est la partie aérienne de la plante, c'est-à-dire l'ensemble des feuilles, fruits, pellicule (c'est-à-dire la peau), pépins et bois, encore plus préférentiellement la peau (pellicule) et les pépins. L'association de la peau, qui peut être riche en resvératrol, et de pépins, qui peuvent être riches en monomères de flavanols, en oligomères de procyanidines et en proanthocyanidines, peut être particulièrement intéressante pour l'invention.

Préférentiellement l'extrait de *Vitis vinifera* est un extrait présentant une teneur en polymères de flavanols inférieure à 0,5% en poids du poids total des polyphénols de l'extrait, encore plus préférentiellement une teneur inférieure à 0,1%. Par « polymère de flavanol » on entend un flavanol présentant un degré de polymérisation supérieur à 10. Selon l'invention, les polymères de flavanols sont très peu biodisponibles à l'inverse des monomères de flavanols qui eux sont très rapidement absorbés dans l'intestin grêle puis métabolisés en dérivés méthylés, sulfatés, et glucuronidés. Cette faible présence de polymères est un critère de qualité des extraits de raisin utilisés en particulier pour l'efficacité et la biodisponibilité.

Par « extrait de *Vaccinium angustifolium »* au sens de l'invention on entend au moins une molécule, préférentiellement un ensemble de molécules, obtenue(s) à partir de *Vaccinium angustifolium.* La matière première peut être les feuilles et/ou les fruits, préférentiellement la matière première est l'ensemble des feuilles et fruits de la plante.

Par extrait obtenu à partir d'un mélange de *Vitis vinifera* et de *Vaccinium angustifolium* on entend un ensemble de molécules obtenues à partir d'un mélange de *Vitis vinifera* et de *Vaccinium angustifolium.* La matière première de *Vitis vinifera* peut être les feuilles et/ou les fruits et/ou les pépins et/ les bois, préférentiellement la matière première de *Vitis vinifera* est la partie aérienne de la plante, c'est-à-dire l'ensemble des feuilles, fruits, peau (péllicule), pépins et bois, plus préférentiellement la peau (pellicule) et les pépins. La matière première de *Vaccinium angustifolium* peut être les feuilles et/ou les fruits, préférentiellement la matière première de *Vaccinium angustifolium* est l'ensemble des feuilles et fruits de la plante. Les extraits selon l'invention peuvent être obtenus par tout procédé permettant d'obtenir un mélange comprenant :
- au moins 1% de catéchine et/ou épicatéchine en poids par rapport au poids total du mélange, préférentiellement au moins 5%, encore plus préférentiellement entre 5% et 50%, notamment entre 7% et 35%,
- au moins 5 ppm d'acide férulique, préférentiellement au moins 10 ppm, encore plus préférentiellement entre 5 ppm et 300 ppm, notamment entre 10 ppm et 100 ppm,
- au moins 200 ppm de resvératrol, préférentiellement au moins 300 ppm, encore plus préférentiellement au moins 400 ppm, encore plus préférentiellement entre 300 ppm et 6000 ppm, notamment entre 400 et 6000 ppm,
- au moins 50 ppm de quercétine et/ou glycosides de quercétine, préférentiellement au moins 70 ppm de quercétine et/ou glycoside, notamment entre 50 ppm et 10000 ppm,
- au moins 500 ppm d'anthocyanidines, préférentiellement au moins 600 ppm, encore plus préférentiellement au moins 700 ppm, encore plus préférentiellement entre 600 ppm et 5000 ppm,
- au moins 300ppm de la malvidine 3 glucoside, la malvidine 3 glucoside étant l'anthocyanidine prépondérante.

De préférence, les anthocyanidines comprennent au moins 20%, de préférence encore au moins 25% de malvidine 3 glucoside (pourcentage en poids).

Un procédé particulièrement adapté est un procédé comprenant les étapes suivantes :
- obtention d'un extrait de *Vitis vinifera :*
   ∘ extraction à l'eau et/ou à l'éthanol de *Vitis vinifera,* préférentiellement de l'ensemble des feuilles, fruits, pellicule, pépins et bois de *Vitis vinifera,*
      La quantité de solvant (30% v/v à 96% V/V) mise en oeuvre est comprise entre 2 et 10 fois la masse de matière mise en œuvre. La durée de l'extraction peut être comprise entre 30 minutes et 24 heures et la température d'extraction comprise entre 20°C et 80°C. Les matières premières utilisées peuvent être sous formes sèches, fraîches, ou congelées entières ou broyées ;
   ∘ séparation de la solution d'eau et/ou éthanol de la matière solide, par exemple par décantation centrifuge ou par pressage et filtration ;
   ∘ évaporation de l'éthanol par évaporation sous-vide à une température préférentiellement inférieure à 60°C et à une pression inférieure à 100mbars ;
   ∘ séparation membranaire de l'extrait préalablement désolvanté de façon à sélectionner préférentiellement les monomères et les oligomères proanthocyanidiques (ayant un degré de polymérisation compris entre 2 et 10 inclus) et d'éliminer les polymères de flavanols (> au décamères), pour obtenir un extrait caractérisé par une teneur en polymères de flavanols inférieure à 0,5% et plus préferentiellement inférieure à 0,1% en poids par rapport au poids total des polyphenols de l'extrait. Cette étape peut être réalisée à l'aide d'une membrane de filtration ayant un seuil de coupure inférieur à 15 000 daltons et plus préférentiellement inférieur à 3000 daltons ;
- obtention d'un extrait de *Vaccinium angustifolium* :
   ∘ extraction à l'eau et/ou à l'éthanol de *Vaccinium angustifolium,* préférentiellement de l'ensemble des feuilles et fruits de *Vaccinium angustifolium,* La quantité de solvant (30% v/v à 96% V/V) mise en oeuvre est comprise entre 2 et 10 fois la masse de matière mise en œuvre. La durée de l'extraction peut être comprise entre 30minutes et 24heures et la température d'extraction comprise entre 20°C et 80°C. Les matières premières utilisées peuvent être sous formes sèches, fraîches, ou congelées ;
   ∘ séparation de la solution d'eau et/ou éthanol de la matière solide par décantation centrifuge ou par pressage et filtration ;
   ∘ évaporation de l'éthanol par évaporation sous-vide à une température préférentiellement inférieure à 60°C et une pression inférieure à 100mbars ;
- séchage des extraits par atomisation, étuve sous vide ou lyophilisation avec ou sans support comme une maltodextrine ;
- mélange de l'extrait de *Vitis vinifera* et de *Vaccinium angustifolium* avant ou après l'étape de séchage.

Selon une variante (hors invention), le procédé consiste en la mise en œuvre des étapes suivantes :
∘ mélange de de *Vitis vinifera* et de *Vaccinium angustifolium* extraction à l'eau et/ou à l'éthanol de *Vaccinium angustifolium,* préférentiellement de l'ensemble des feuilles et fruits de *Vaccinium angustifolium,* La quantité de solvant (30% v/v à 96% V/V) mise en oeuvre est comprise entre 2 et 10 fois la masse de matière mise en œuvre. La durée de l'extraction peut être comprise entre 30minutes et 24heures et la température d'extraction comprise entre 20°C et 80°C. Les matières premières utilisées peuvent être sous formes sèches, fraîches, ou congelées ;
∘ séparation de la solution d'eau et/ou éthanol du marc solide par décantation centrifuge ou par pressage et filtration ;

- évaporation de l'éthanol par évaporation sous-vide à une température préférentiellement inférieure à 60°C et une pression inférieure à 100mbars ;
- séchage de l'extrait par pulvérisation ou sublimation avec ou sans support comme une maltodextrine.

Quelle que soit la variante du procédé, avant l'étape de séchage, le procédé peut comprendre les étapes suivantes :
- chargement sur une résine des solutions d'extraits mélangées ou non,
- rinçage de la résine avec de l'eau,
- application d'une solution éluante eau/éthanol sur la résine,
- récupération de l'éluat purifié,
- évaporation de l'éthanol dudit éluât,
- concentration dudit éluât
- séchage dudit extrait aqueux purifié.

L'agent nutritionnel ou thérapeutique selon l'invention peut être constitué exclusivement du mélange de molécules, c'est-à-dire des extraits, ou comprendre d'autres constituants. Préférentiellement, en plus du mélange de molécules, l'agent nutritionnel ou thérapeutique selon l'invention contient d'autres constituants, en particulier des excipients ou des agents d'enrobage, tels que de la maltodextrine, de la cellulose microcristalline, des cyclodextrines, de l'amidon, des fibres solubles ou insolubles.

L'agent peut se présenter sous toute forme adaptée à une application nutritionnelle ou thérapeutique, préférentiellement sous forme de poudre.

L'agent selon l'invention peut être intégré dans une composition, en particulier dans une composition nutritionnelle ou thérapeutique (médicament) se présentant sous une forme choisie parmi les comprimés, les capsules, les gélules, les poudres, les solutions, les microcapsules, les suspensions, les émulsions, les compléments alimentaires, les boissons, et les aliments pour l'Homme ou l'animal.

Il peut s'agir d'une composition nutritionnelle non thérapeutique destinée à l'Homme comme par exemple des compléments alimentaires, des barres, des produits laitiers, des poudres à avaler ou à réhydrater, des gels, des confitures, des bonbons, des boissons gazeuses ou non, des boissons sèches à réhydrater, des compotes.

Il peut s'agir également d'un médicament destiné à l'homme comme par exemple des comprimés ou des gélules.

Il peut s'agir aussi d'une composition nutritionnelle ou thérapeutique destinée à l'animal. On entend par « animal » tout animal pouvant recevoir un agent nutritionnel ou thérapeutique selon l'invention, par exemple mais de manière non limitative un animal de compagnie, une volaille, un porc, un ruminant, un caprin, ou encore une souris. De manière préférentielle, l'animal est un animal de compagnie, tel que le chat ou le chien. De préférence encore, l'animal est un chien.

Il peut s'agir aussi d'une composition nutritionnelle non thérapeutique destinée à l'animal comme par exemple des aliments secs, tels que des croquettes (extrudées, co-extrudées ou lyophilisées), des friandises (ou treats), des snacks, des aliments humides ou semi-humides tels que des morceaux en sauce, des morceaux en gelée, des boissons, ou encore des compléments alimentaires. De préférence, l'agent est intégré dans des aliments secs tels que des croquettes.

Il peut s'agir enfin d'un médicament destiné à l'animal, ou produit vétérinaire, comme par exemple des comprimés, des gélules, des spray, des liquides administrés par gouttes

De manière avantageuse, l'agent destiné à l'animal peut être intégré dans une composition, en particulier dans une composition nutritionnelle ou thérapeutique, en inclusion, c'est-à-dire en l'ajoutant dans la masse de la composition par exemple par imprégnation ou mélange, ou en enrobage, c'est-à-dire en en l'appliquant en surface de la composition, par pulvérisation ou par saupoudrage, par exemple en le mélangeant préalablement à un ou plusieurs ingrédients tels que au moins un facteur d'appétence.

L'agent nutritionnel ou thérapeutique peut être utilisé en particulier pour agir sur les fonctions cognitives et exécutives chez un individu ou un animal sain mais également chez des sujets malades.

Le déclin cognitif est caractérisé par une diminution liée à l'âge des fonctions cognitives et exécutives en particulier de la concentration, du travail, de la mémoire à long terme, des capacités de raisonnement, de jugement, de résolution de problèmes et de vitesse de traitement des informations. Ces déficiences peuvent conduire à une diminution de l'estime de soi et de la qualité de vie. Le déclin cognitif lié à l'âge est le terme utilisé pour décrire la forme non pathologique de la détérioration de la mémoire et des fonctions cognitives conséquente au processus de vieillissement dans les limites normales, compte-tenu de l'âge d'une personne. Il s'agit d'un processus complexe, avec des premiers signes émergeant chez l'Homme entre 35 et 65 ans, sans lésions neurodégénératives spécifiques. Le déclin cognitif progressif est perceptible par l'apparition de problèmes cognitifs mineurs qui affectent 15 à 20% de la population âgée de 65 ans ou plus, mais qui représentent un état instable. Toutefois, certaines formes pathologiques peuvent survenir en plus de ce déclin cognitif « normal ». Parmi ces pathologies, la maladie d'Alzheimer est la cause la plus commune de démence, qui touche plus de 24 millions de personnes dans le monde. Elle est irréversible dans notre état actuel des connaissances, les seuls traitements disponibles étant purement symptomatiques. Chez les animaux, ces pathologies peuvent se présenter de manière très similaire. Chez le chien par exemple, le syndrome de dysfonctionnement cognitif (ou SDC) est une pathologie largement répandue et caractérisée par une désorientation spatio-temporelle, une perte des apprentissages élémentaires qui induit souvent de la malpropreté, une altération des cycles veille-sommeil, une altération des interactions sociales.

L'agent selon l'invention est capable d'améliorer les fonctions cognitives et exécutives chez l'Homme ou l'animal. L'association des deux matières premières et la spécificité des extraits selon l'invention comportant des polyphénols combinés dans des quantités particulières, produit un effet synergique en comparaison aux polyphénols pris isolément ou aux extraits existants comprenant ces polyphénols dans des proportions et quantités différentes. La synergie porte sur l'effet antioxydant et/ou sur l'amélioration des fonctions cognitives et/ou exécutives chez l'Homme ou l'animal.

Les polyphénols présents dans l'agent selon l'invention, lorsqu'ils sont administrés à l'Homme ou l'animal, ont également une biodisponibilité améliorée en comparaison aux polyphénols pris isolements ou aux extraits existants comprenant ces polyphénols dans des proportions et quantités différentes. L'agent selon l'invention utilisé chez l'Homme ou l'animal, permet ainsi améliorer la biodisponibilité des polyphénols contenus dans ledit agent.

L'agent selon l'invention peut être utilisé comme médicament pour l'Homme ou l'animal.

L'agent thérapeutique ou nutritionnel pourrait être utilisé dans le traitement ou la prévention chez l'Homme ou l'animal de la maladie d'Alzheimer et/ou de la maladie de Parkinson et/ou de la maladie de Huntington et/ou du déclin cognitif pathologique et/ou de la démence et/ou de la dépression et/ou du diabète et/ou de la schizophrénie et/ou du retard mental et/ou de troubles liés à l'état de post ménopause chez la femme et/ou le syndrome de dysfonctionnement cognitif (CDS).

L'agent selon l'invention peut également être utilisé chez l'Homme ou l'animal sain, pour une utilisation non thérapeutique, pour améliorer les fonctions cognitives et/ou les fonctions exécutives, et/ou pour limiter le déclin cognitif non pathologique lié à l'âge, préférentiellement dans une composition nutritionnelle ou un complément alimentaire. Il pourrait notamment être utilisé chez l'Homme ou l'animal sain pour améliorer la mémoire et/ou l'attention et/ou la concentration et/ou la vivacité et/ou l'apprentissage et/ou l'intelligence et/ou le langage et/ou l'humeur et/ou le stress et/ou l'anxiété et/ou la vision et/ou le sommeil.

Selon un mode de réalisation particulier de l'invention, l'Homme ou l'animal est âgé. De préférence, l'Homme ou l'animal âgé est un Homme ou un animal qui a atteint au moins 50% de la durée de vie moyenne liée à son espèce.

Préférentiellement, l'agent thérapeutique ou nutritionnel est utilisé à une dose, une quantité permettant de fournir à l'Homme ou l'animal malade pour les utilisations thérapeutiques ou sain pour les utilisation non thérapeutiques :
- au moins 100 µg par kg de poids corporel de catéchine et/ou épicatéchine,
- préférentiellement au moins 0,05 µg par kg de poids corporel d'acide férulique,
- au moins 10 µg par kg de poids corporel de resvératrol,
- au moins 0,2 µg par kg de poids corporel de quercétine et/ou de glycosides quercétine, et
- au moins 1 µg par kg de poids corporel d'anthocyanidines.

L'invention est à présent illustrée à travers des exemples et résultats d'essais démontrant l'effet synergique antioxydant et sur les fonctions cognitives et executives, et l'amélioration de la biodisponibilité de l'agent thérapeutique ou nutritionnel objet de la présente demande.

### EXEMPLES

### Exemple 1 : Agent thérapeutique ou nutritionnel selon l'invention

Ce premier exemple de mélange selon l'invention est obtenu par la mise en œuvre du procédé tel que décrit en suivant.

Les matières premières utilisées sont :
- la peau et les graines (pépins) de fruits de *Vitis vinifera,* sous-produits de l'industrie vinicole,
- des baies congelées de *Vaccinium angustifolium.*

400 g de baies congelées de *Vaccinium angustifolium* sont broyées et mélangées avec une solution de 2000ml d'éthanol 80% (V/V) avec une teneur de 0,1% en poids de HCl. Le mélange est maintenu à température ambiante (20°c) pendant 24 heures. La solution éthanolique est ensuite séparée de la pulpe grâce à une filtration, et concentrée sous vide avec un évaporateur rotatif à 20% de matière sèche. Une partie de cet extrait est conservée pour être testée, l'autre partie est conservée pour être mélangée à l'extrait de *Vitis vinifera.*

500 g de pélicule et de pépins de *Vitis vinifera* sont mélangés avec 2500 ml d'éthanol 80% (V/V) avec une teneur de 0,1% en poids de HCl à 40°C pendant 5 heures. La solution éthanolique est ensuite séparée de la pulpe grâce à une filtration. L'éthanol est ensuite éliminé sous vide avec un évaporateur rotatif à une température de 50°C sous 60mbars. La solution aqueuse est ensuite diluée pour avoir une matière sèche de 5% et filtrée sur une membrane de 5000 daltons. Le perméat obtenu est alors chargé sur une colonne de résine (C18) à 1BV/heure. La résine est ensuite rincée une première fois avec 3BV d'eau distillée à 2BV/heure, et ensuite éluée avec 5BV d'une solution éthanolique à 80% (V/V) à 1 BV/heure. Une partie de la solution extraite est conservée pour être testée et caractérisée (Tableau 1a). Les polyphénols présentés dans ce tableau ont été mesurés par chromatographie liquide ultra rapide avec un détecteur fluorescence.

**Tableau 1a : Contenu en flavanols de l'extrait de Vitis vinifera**

| Teneur en monomères de flavanols et en proanthocyanidines (% de poids sec - eq. Epicatechines) (mesuré par LC avec un détecteur fluorescence) | |
|---|---|
| Monomères | 35,27% ± 1,67 |
| Dimeres | 22,92% ± 1,31 |
| Trimères | 8,93% ± 0,46 |
| Tétramères | 2,95% ±0,14 |
| Pentamères | 1,09% ± 0,07 |
| Hexamères | 0,63% ±0,14 |
| Heptamères | 0,15%± 0,05 |
| Octamères | Non Détécté |
| Nonamères | Non Détécté |
| Décamères | Non Détécté |
| Polymères (Degré de polymérisation >10) | Non Détécté |

L'autre partie est ensuite mélangée avec l'extrait de *Vaccinium angustifolium* pour former le mélange selon l'invention et une maltodextrine est ajoutée au mélange jusqu'à obtenir une solution présentant un taux de matière sèche de 30%.

La solution est ensuite séchée par pulvérisation avec une température d'entrée de 160°C.

Le produit obtenu est une poudre mauve contenant les polyphénols présentés dans le tableau 1b. Les polyphénols présentés dans ce tableau ont été mesurés par chromatographie liquide ultra rapide UPLC-MS/MS.

**Tableau 1b : contenu en polyphénols de l'agent selon l'invention de l'exemple 1**

| | Mélange selon l'invention | 500mg de mélange/kg de poids corporel souris | Equivalent en mg/kg (Homme selon la FDA) |
|---|---|---|---|
| Catéchine et épicatéchine | 25,7% | 128,5 mg/kg de poids corporel | 10,4 mg/kg de poids corporel |
| Anthocyanidines (dont malvidine 3 glucoside) | 0,436% (1310ppm) | 2,18 mg/kg de poids corporel | 177µg/kg de poids corporel |
| Quercetine et glycosydes de quercétine | 0,864% | 4,32 mg/kg de poids corporel | 35µg/kg de poids corporel |
| Acide férulique | 0,0094% | 47µg /kg de poids corporel | 3,82µg/kg de poids corporel |
| Resvératrol | 0,0437% | 218 µg/kg de poids corporel | 17,7µg/kg de poids corporel |

### Exemple 2 : Agent thérapeutique ou nutritionnel selon l'invention

Ce deuxième exemple de mélange selon l'invention est obtenu par la mise en œuvre du procédé tel que décrit en suivant.

Les matières premières utilisées sont :
- les pépins et la peau de *Vitis vinifera,*
- des baies de *Vaccinium angustifolium.*

1000g de marc congelé de *Vaccinium angustifolium* sont broyés et mélangés avec une solution de 5000ml d'éthanol 60% (V/V) avec une teneur de 0,1% en poids de HCl. Le mélange est maintenu à température ambiante (20°c) pendant 24 heures. La solution éthanolique est ensuite séparée de la pulpe grâce à une filtration, et concentrée sous vide avec un évaporateur rotatif à 20% de matière sèche.

400 g de peau et de pépins de *Vitis vinifera* sélectionnés sont mélangés avec 1500 ml d'éthanol 80% (V/V) à 60°C pendant 5 heures. La solution éthanolique est ensuite séparée de la pulpe grâce à une filtration. L'éthanol est ensuite éliminé sous vide avec un évaporateur rotatif à une température de 50°C sous 60mbars. La solution aqueuse est ensuite diluée pour avoir une matière sèche de 5% et filtrée sur une membrane de 5000 daltons. Le perméat obtenu est alors chargé sur une colonne de résine (C18) à 1BV/heure. La résine est ensuite rincée une première fois avec 3BV d'eau distillée à 2BV/heure, et ensuite éluée avec 5BV d'une solution éthanolique à 80% (V/V) à 1 BV/heure.

Une partie de la solution extraite est conservée pour être testée et caractérisé (tableau 2a) ,

**Tableau 2a : contenu en flavanols de l'extrait de Vitis vinifera**

| Teneur en monomères de flavanols et en proanthocyanidines (% de poids sec - eq. Epicatechines) (mesuré par LC avec un détecteur fluorescence) | |
|---|---|
| Monomères | 9,4% ± 0,8 |
| Dimeres | 4,0% ± 0,3 |
| Trimères | 0,81% ± 0,1 |
| Tétramères | 0,24% ± 0,1 |
| Pentamères | Non Détécté |
| Hexamères | Non Détécté |
| Heptamères | Non Détécté |
| Octamères | Non Détécté |
| Nonamères | Non Détécté |
| Décamères | Non Détécté |
| Polymères (Degré de polymérisation >10) | Non Détécté |

Le produit obtenu est une poudre mauve contenant les polyphénols présentés dans le tableau 2b. Les polyphénols présentés dans ce tableau ont été mesurés par chromatographie liquide ultra rapide UPLC-MS/MS.

**Tableau 2b : contenu en polyphénols de l'agent selon l'invention de l'exemple 2**

| | Mélange selon l'invention | 4mg de mélange/kg poids corporel souris | Equivalent en mg/kg (Homme selon la FDA) |
|---|---|---|---|
| Catéchine et épicatéchine | 9,4% | 376 µg/ kg de poids corporel | 124 µg/ kg de poids corporel |
| Anthocyanidines (dont malvidine 3 glucoside) | 700ppm (328 ppm) | 2.8 µg/ kg de poids corporel | 1.6µg/ kg de poids corporel |
| Quercetine et glycosydes de quercétine | 77ppm | 0.37µg/ kg de poids corporel | 0.21µg/ kg de poids corporel |
| Acide férulique | 20ppm | 0.08 µg/ kg de poids corporel | 0.044µg/ kg de poids corporel |
| Resvératrol | 5327ppm | 21 µg/ kg de poids corporel | 12µg/ kg de poids corporel |

### Exemple 3 : Composition thérapeutique ou nutritionnel selon l'invention

19,980 kg de l'agent de l'exemple 1 est mélangé avec 0,020kg de Silice colloidale. La composition est obtenue par mélange des constituants dans les conditions classiques connues de l'homme du métier. L'agent est conditionné dans un sac PET lui-même conditionné dans un carton.

### Exemple 4: Composition nutritionnelle destinée à l'homme

L'exemple 4 est une gélule de 400mg, constituée par :
- Agent thérapeutique de l'exemple 1 : 300mg
- Vitamine C : 80mg
- Maltodextrine : 20mg

La composition est obtenue par mélange des constituants dans les conditions classiques connues de l'homme du métier, et mis dans une gélule selon les conditions classiques également.

La posologie préconisée est de 1 à 2 gélules par jour.

### Exemple 5 : Médicament destiné à l'homme

L'exemple 5 est un comprimé de 3 000mg, constitué par :
- Agent thérapeutique de l'exemple 1 : 1500mg
- Sorbitol : 1400mg
- Arôme fruit rouge : 47 mg
- Stéarate de magnesium : 30mg
- Colorant bleu brillant FCF laque E133 : 20mg
- Acesulfame K (E950) : 1,5mg
- Saccharinate de sodium (E954) : 1,5mg

La composition est obtenue par mélange des constituants dans les conditions classiques connues de l'homme du métier.

La posologie préconisée est de 1 à 2 comprimés par jour.

### Exemple 6 : Composition nutritionnelle animale

L'agent selon l'invention de l'exemple 2 a été ajouté à une croquette sèche extrudée pour chien respectant les normes de l'AFCO et comprenant des farines animales, de la matière grasse, des fibres, des céréales et des agents conservateurs et antioxydants.

L'ajout de l'agent à la croquette a été fait selon plusieurs modes de réalisation, notamment par enrobage et par inclusion.

Des essais en enrobage ont été effectués en ajoutant l'agent selon l'invention à un facteur d'appétence liquide D'Tech volaille (SPF, Elven, France). Une première couche de matière grasse de volaille (6% par rapport au poids de la croquette) a été ajoutée en enrobage sur la croquette, suivie par une couche de mélange entre le facteur d'appétence (1%, 2% ou 3%, le % étant par rapport au poids de la croquette) et l'agent selon l'invention (0,02%, 0,04% ou 0,1%, le % étant par rapport au poids de la croquette).

Des essais en inclusion ont été effectués en ajoutant l'agent selon l'invention (0,02%, 0,04% ou 0,1%, le % étant par rapport au poids de la croquette) dans la matière première (aussi appelé prémix) avant extrusion.

### Exemple 7 : Produit vétérinaire

Des capsules en gélatine ont été préparées de manière standard, en ajoutant un agent selon l'invention de l'exemple 2 et une maltodextrine (Control; Glucidex12 lot#421A323532, Roquette, Lestrem Cedex, France).

### EVALUATION DE L'EFFICACITE ET DE LA BIODISPONIBILITE DE L'AGENT SELON INVENTION

### Essai 1 : Effet sur la biodisponibilité chez des souris

L'objectif de cet essai est de comparer la biodisponibilité des polyphénols contenus dans l'agent thérapeutique ou nutritionnel selon l'invention (mélange de l'exemple 1) avec la biodisponibilité des polyphénols contenus dans un extrait de *Vitis viniferaVitis vinifera* et de ceux contenus dans un extrait de *Vaccinium angustifolium* (ceux décrits dans l'exemple 1), après une administration orale aigüe (1 jour) et chronique (15 jours) à des souris.

Soixante-douze souris mâles et femelles âgées de 4 mois ont été divisées en deux groupes pour réaliser l'étude aigüe et l'étude chronique séparément. Dans chaque groupe, trois sous-groupes de 10 ont été créés, chaque sous-groupe recevant un traitement différent : extrait de *Vitis vinifera,* extrait de *Vaccinium angustifolium* et mélange de l'exemple 1, et un quatrième sous-groupe de 6 souris traités avec de l'eau (groupe contrôle). Les traitements sont administrés par voie orale par gavage. Le mélange a été administré à une dose de 500 mg/kg de poids corporel, et l'extrait de *Vitis vinifera* et l'extrait de *Vaccinium angustifolium* ont été administrés à une dose équivalente à leur quantité dans la dose de mélange.
- étude aigüe : des échantillons de sang sont prélevés pour chaque groupe, avant le gavage puis 30 minutes après le gavage pour chaque traitement respectif. Les animaux sont ensuite sacrifiés et, des échantillons de sang sont également prélevés.
- étude chronique : des échantillons de sang sont collectés avant la supplémentation (jour 0). Les animaux reçoivent ensuite leurs traitements respectifs chaque jour, pendant 15 jours. Le dernier jour de l'étude (jour 15), 30 minutes après gavage les animaux sont sacrifiés et des échantillons de sang sont collectés. Les excréments sont collectés pour chaque souris avant et pendant la période de supplémentation (jour 0 et jours 1 à 15 respectivement).

Les métabolites phénoliques sont extraits des échantillons de plasma et des excréments secs par micro extraction en phase solide (µSPE) et caractérisés par UHPLC-MS/MS (chromatographie liquide ultra rapide). Les concentrations plasmatiques des métabolites phénoliques après administration aigüe et chronique des différents traitements, ont été comparées en utilisant le test statistique de Welch (correction de variance inégale) quand les données étaient supposées être normalement distribuées, ou quand ce n'était pas le cas en utilisant le test de Mann-Whitney, avec le logiciel GraphPad Prism 6.05. De la même façon, les effets des traitements sur les concentrations des métabolites phénoliques circulants et les concentrations accumulées dans les excréments ont été analysés pour la comparaison par paires en utilisant le test statistique de Welch pour les données normales et le test de Mann-Whitney autrement.

Les comparaisons multiples ont été réalisées en utilisant une analyse de variance (ANOVA) ou le test non paramétrique de Kruskal-Wallis, test basé sur des données suivant une distribution normale ou non. Les différences ont été considérées comme significatives à p<0.05.

La classification ascendante hiérarchique (CAH) des métabolites phénoliques détectés dans le plasma et les excréments de souris a été effectuée en utilisant MetaboAnalyst 3.0.

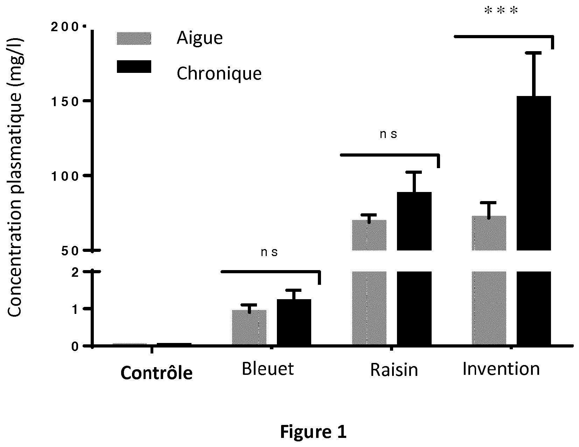

Les résultats sont présentés sur les figures 1 à 3.

La Figure 1 représente les différences de biodisponibilité des composés phénoliques entre une administration aigüe et chronique des traitements. Les résultats sont donnés sous forme de moyenne ± SEM. *** P < 0,005 versus supplémentation aigüe. Le terme « ns » sur la figure signifie non significatif.

On constate sur cette Figure 1, qu'aucune différence n'est observée dans les concentrations des métabolites phénoliques circulants entre la supplémentation aigüe et la supplémentation chronique pour les traitements avec l'extrait de *Vitis vinifera* (raisin) et avec l'extrait de *Vaccinium angustifolium* (bleuet).

Par contre, la supplémentation répétée selon l'invention, i.e. extrait de *Vitis vinifera* et extrait de *Vaccinium angustifolium,* pendant 15 jours est associée à une augmentation de la concentration en composés phénoliques dans le plasma (de 2,1 fois, p = 0,0033) par rapport aux souris ne recevant qu'une dose unique.

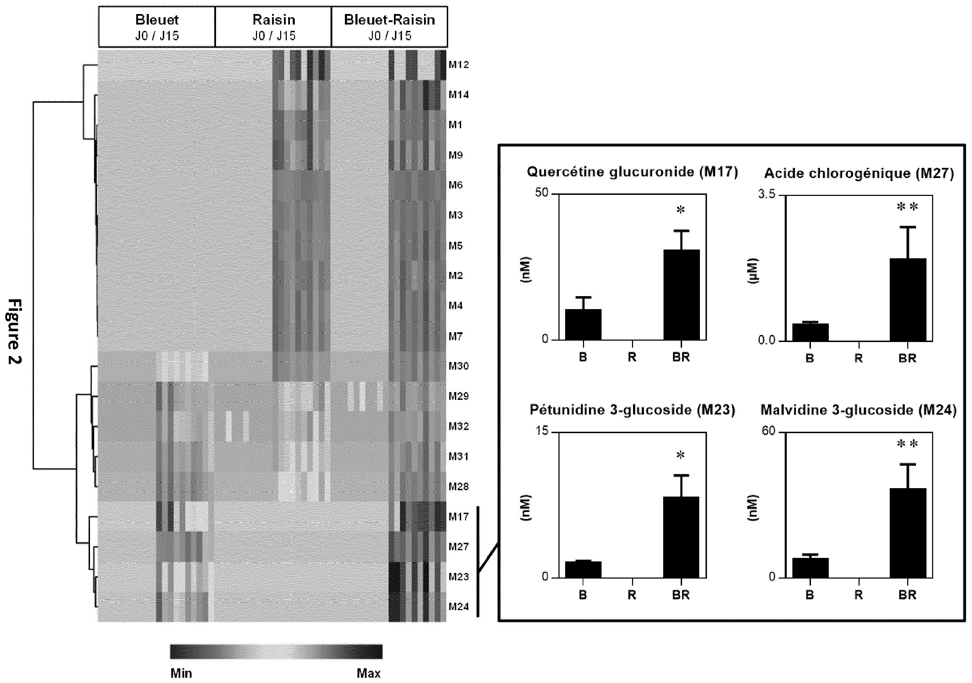

La Figure 2 représente la classification ascendante hiérarchique (CAH) des métabolites phénoliques analysés chez les souris avant (jour 0) et après (jour 15) l'ingestion chronique des trois traitements.

Chaque ligne correspond à un métabolite détecté et chaque colonne à un animal étudié. Les carreaux en nuances de gris indiquent l'intensité de la concentration en métabolites dans le plasma par rapport à la moyenne de tous les échantillons. Les graphiques encadrés représentent les métabolites phénoliques de l'extrait de *Vaccinium angustifolium* dont la concentration dans le plasma a été augmentée de façon significative avec le traitement selon l'invention. Les données sont affichées sous forme de moyenne ± SEM. ** P <0,01 et p * <0,05 vs extrait de *Vaccinium angustifolium* B: extrait de *Vaccinium angustifolium.* G: extrait de *Vitis vinifera,* N: Agent selon l'invention.

Comme visualisé sur la carte (heat map), on constate qu'il n'y a pas de différence dans la concentration des métabolites phénoliques circulants provenant de *Vitis vinifera* entre une supplémentation avec l'extrait de *Vitis vinifera* (raisin) et une supplémentation avec l'agent selon l'invention, alors que les métabolites phénoliques provenant de *Vaccinium angustifolium* ont été trouvés de façon plus importante dans le plasma des souris lors de la supplémentation avec l'agent selon l'invention que lors de la supplémentation avec l'extrait de *Vaccinium angustifolium* (bleuet). En effet, tel que l'on peut l'observer sur les encadrés, alors que la même quantité d'extrait de *Vaccinium angustifolium* est administrée dans les deux cas, avec l'agent selon l'invention on constate une augmentation de l'absorption des composés phénoliques de *Vaccinium angustifolium* de 3,0 à 5,5 fois. Cette augmentation, bien que plus faible (2,3 à 2,8 fois) a également été constatée après la supplémentation aigüe en agent selon l'invention versus extrait de *Vaccinium angustifolium.*

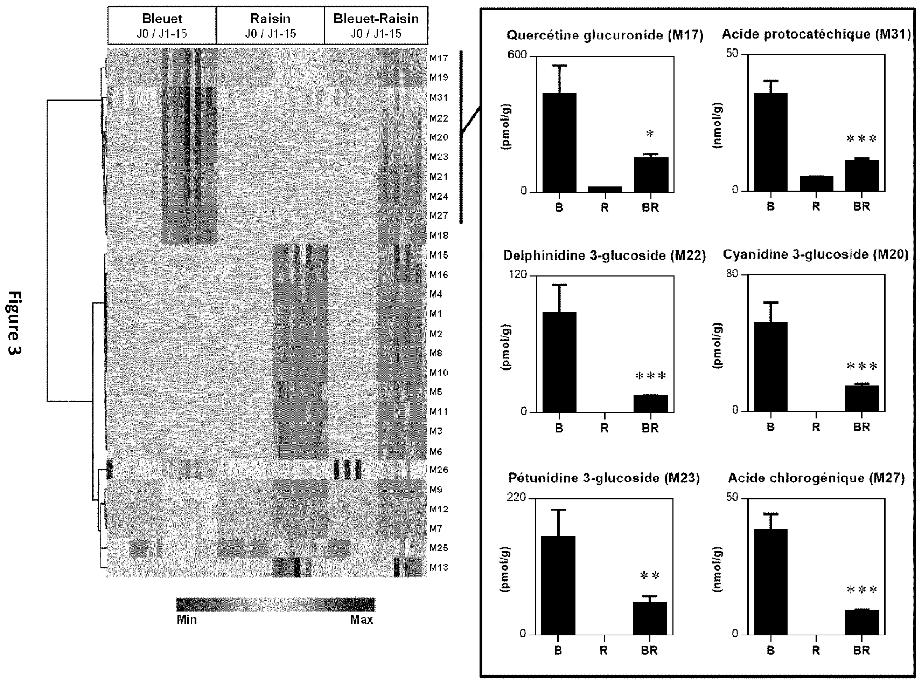

La Figure 3 représente la carte (heat map) CAH des métabolites phénoliques analysés dans les excréments de souris avant (jour 0) et après (jours 1 à 15) l'ingestion chronique d'extrait de *Vaccinium angustifolium* (bleuet), d'extrait de *Vitis vinifera* (raisin), et de l'agent selon l'invention.

Chaque ligne correspond au métabolite détecté et chaque colonne à un animal étudié. Les carreaux en nuances de gris indiquent l'intensité de la concentration en métabolites phénoliques dans les excréments par rapport à la moyenne des échantillons. Les encadrés représentent les métabolites phénoliques de *Vaccinium angustifolium* dont la concentration dans les excréments a été significativement diminuée quand l'agent selon l'invention a été ingéré versus l'extrait de *Vaccinium angustifolium.* Les résultats ont été donnés en moyenne ± SEM. *** p<0.005 and ** p<0.01 versus extrait de *Vaccinium angustifolium* B: extrait de *Vaccinium angustifolium.* G: extrait de *Vitis vinifera,* N: Agent selon invention. Comme observé dans les échantillons de sang , aucune différence n'a été trouvée pour les métabolites phénoliques de *Vitis vinifera* entre la supplémentation avec l'agent selon l'invention et la supplémentation avec l'extrait de *Vitis vinifera,* alors que les métabolites phénoliques de *Vaccinium angustifolium* ont été trouvés à des concentrations significativement plus faibles dans les excréments de souris supplémentées avec l'agent selon l'invention en comparaison avec la supplémentation avec l'extrait de *Vaccinium angustifolium.* En effet, comme montré sur les encadrés, et en accord avec les précédentes observations montrant une augmentation de l'absorption des métabolites phénoliques de *Vaccinium angustifolium* lors que la supplémentation avec l'agent selon l'invention, on constate ici aussi qu'une telle supplémentation est associée à une diminution des excrétions de composés phénoliques de *Vaccinium angustifolium* de 2,9 à 6,3 fois.

Tous ces résultats démontrent qu'il existe des interactions synergiques entre les extraits de *Vaccinium angustifolium* et de *Vitis vinifera* conduisant à une augmentation de la biodisponibilité.

### Essai 2 : biodisponibilité dans le cerveau de souris

Cette étude a pour objet de déterminer si les polyphénols et leurs dérivés métaboliques sont capables d'accéder au système nerveux central, pour savoir s'ils ont des effets dans le cerveau. Pour évaluer la présence de polyphénols dans le cerveau, 6 souris contrôle (3 adultes et 3 âgées) et 20 souris supplémentées (10 adultes et 10 âgées) ont été nourries avec une alimentation contrôlée exempte de polyphénols ou avec une alimentation enrichie avec l'agent selon l'invention (exemple 1) pendant 6 semaines. La dose de l'agent selon l'invention a été de 500mg/kg de poids corporel / jour. Les cerveaux des souris ont été récupérés à la fin de l'expérimentation, disséqués et conservés à -80°C. Les polyphénols spécifiques et les métabolites ont été mesurés par chromatographie liquide ultra rapide *UPLC-MS*/*MS.*

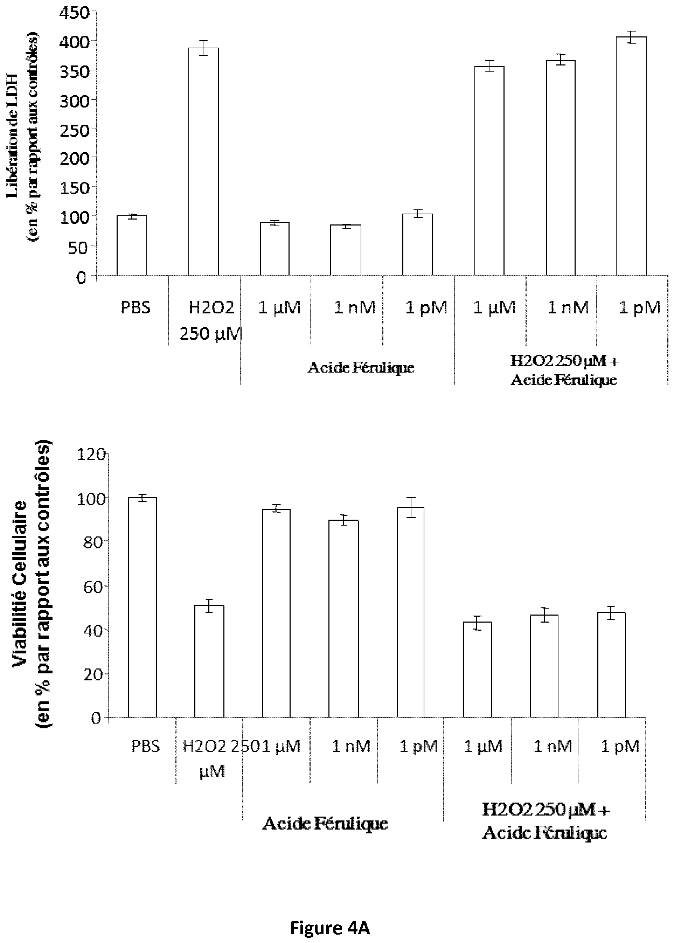

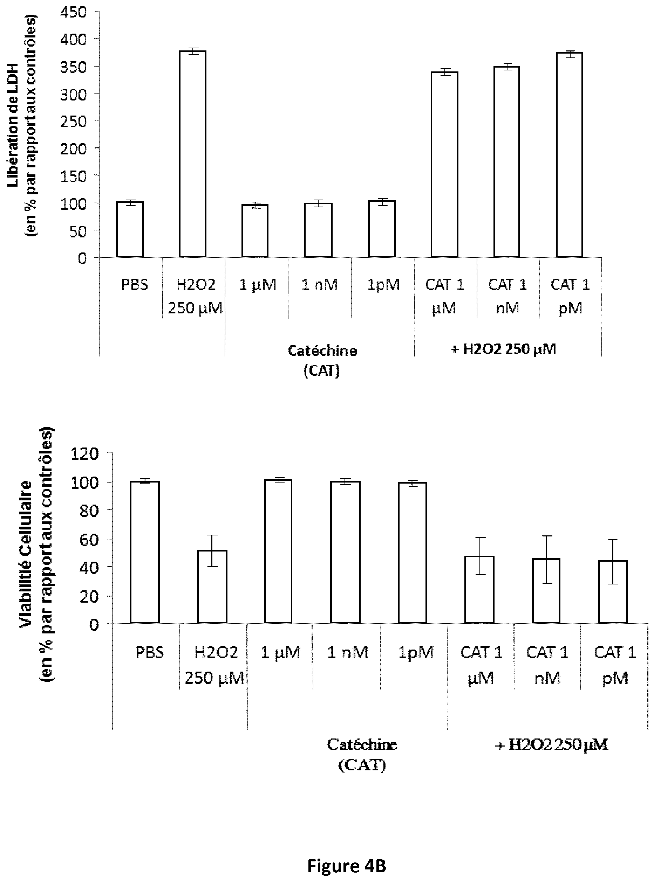

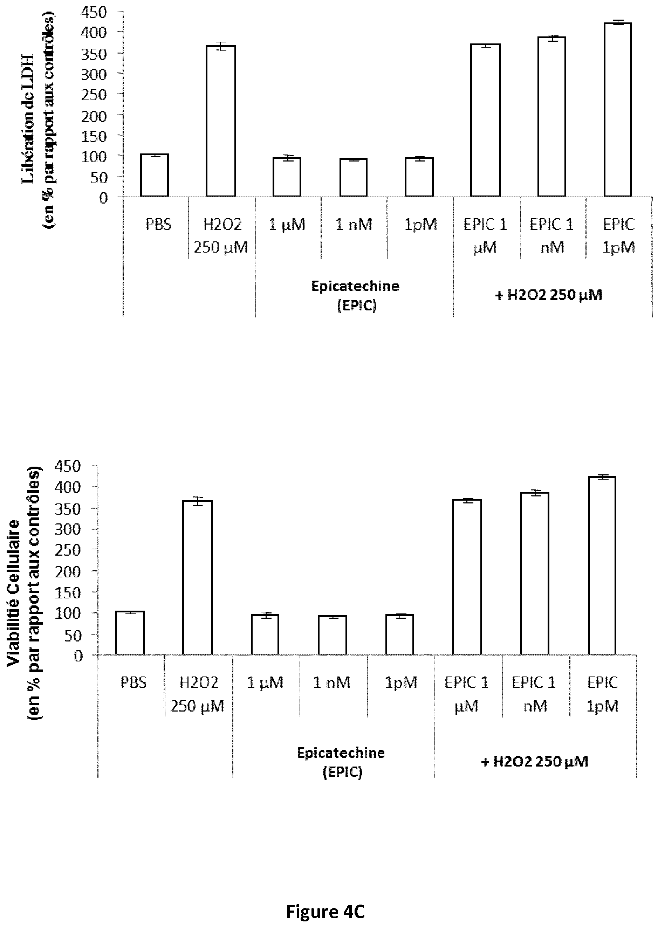

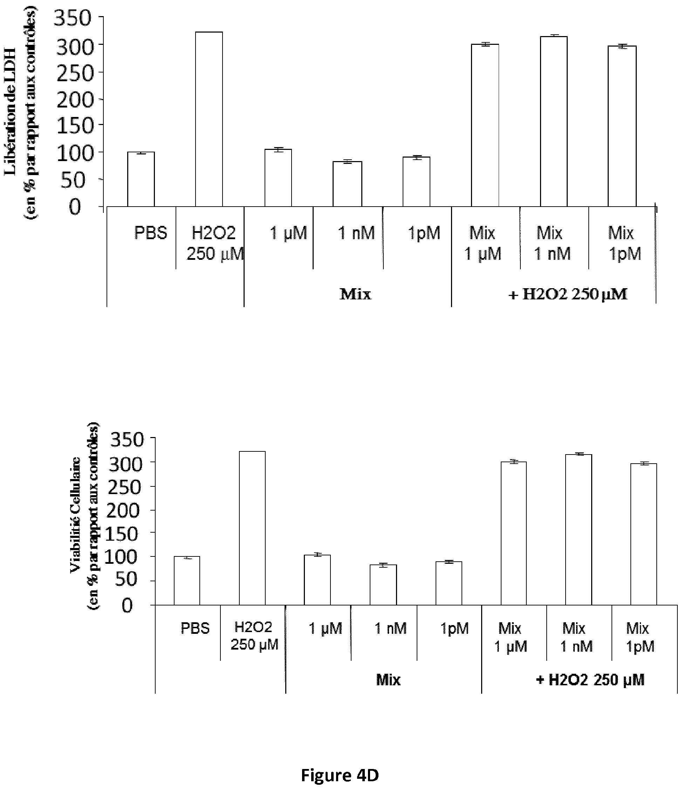

Les résultats sont présentés dans le tableau 3 ci-dessous, et sur les Figures 4A à 4D.

**Tableau 3 : quantité de polyphénols détectés dans le cerveau de souris**

| Contenu du cerveau, pmol/g | Adultes contrôle | Agées contrôle | Adultes Agent selon l'invention | Agées Agent selon l'invention |
|---|---|---|---|---|
| Epicatéchine | 0 | 0 | 3.54±4.99 | 8.84±15.40 |
| Catéchine | 0 | 0 | 0.95±1.45 | 7.12±10.45 |
| PAC Dimers B | 0 | 0 | 2.70±4.52 | 5.82±8.33 |
| Méthyl catéchines glucuronide | 0 | 0 | 5.60±8.56 | 3.15±3.54 |
| Catéchines glucuronide | 0 | 0 | 0.81±1.85 | 1.47±1.88 |
| Acide Férulique | 0 | 0 | 0.80±2.01 | 0.42±0.96 |

Après 6 semaines de consommation d'une alimentation enrichie avec l'agent selon l'invention, des catéchines et épicatéchines et leurs métabolites *(méthyl-catéchine-glucuronide, catéchine-glucuronide)* et de l'acide férulique ont été trouvés dans les cerveaux des souris. Des dimères de proanthocyanidines ont également été trouvés. Ces polyphénols n'ont pas été trouvés dans les cerveaux des souris contrôle. Aucune différence significative n'a été trouvée en fonction de l'âge. Les polyphénols de l'agent selon l'invention, du fait de leur association particulière et de leur quantité spécifique peuvent atteindre directement le cerveau pour produire leurs effets neuroprotecteurs.

### Essai 3 : propriété des polyphénols sur un modèle de culture cellulaire neuronal

Comme les épicatéchine, catéchine et acide férulique ont été détectés dans le cerveau, l'objectif de cette étude est de tester leur capacité à protéger les cellules neuronales et leur effet synergique potentiel en utilisant différents modèles expérimentaux.

Pour cela, les cellules SK-N-SH, une lignée cellulaire de neuroblastes humains, ont été maintenues dans un milieu MEM supplémenté avec 10% (V/V) de FBS, 100 U/ml de pénicilline, 100 µg/ml de streptomycine, et 1% de pyruvate de sodium (1 mM) dans un incubateur humidifié à 37°C avec 5% de CO₂. Les cellules ont été cultivées jusqu'à 80% de confluence, puis ensemencées dans des plaques multi-puits de culture cellulaire pour réaliser différents modèles expérimentaux. L'effet neuroprotecteur de différents composés a été analysé par deux essais différents et complémentaires : le test de quantification de mort cellulaire et le test de survie cellulaire.

Le test de mort cellulaire est un test colorimétrique basé sur la mesure de l'activité du lactate déshydrogénase (LDH) libérée par le cytosol des cellules endommagées dans le surnageant.

Le test de survie des cellules est a été effectué par un test de Résazurine. La résazurine est un indicateur d'oxydo-réduction de la cellule perméable qui peut être utilisé pour surveiller le nombre de cellules viables en utilisant les composés de tétrazolium. Les cellules viables avec un métabolisme actif peuvent réduire la résazurine en produit résorufine qui est rose et fluorescent.

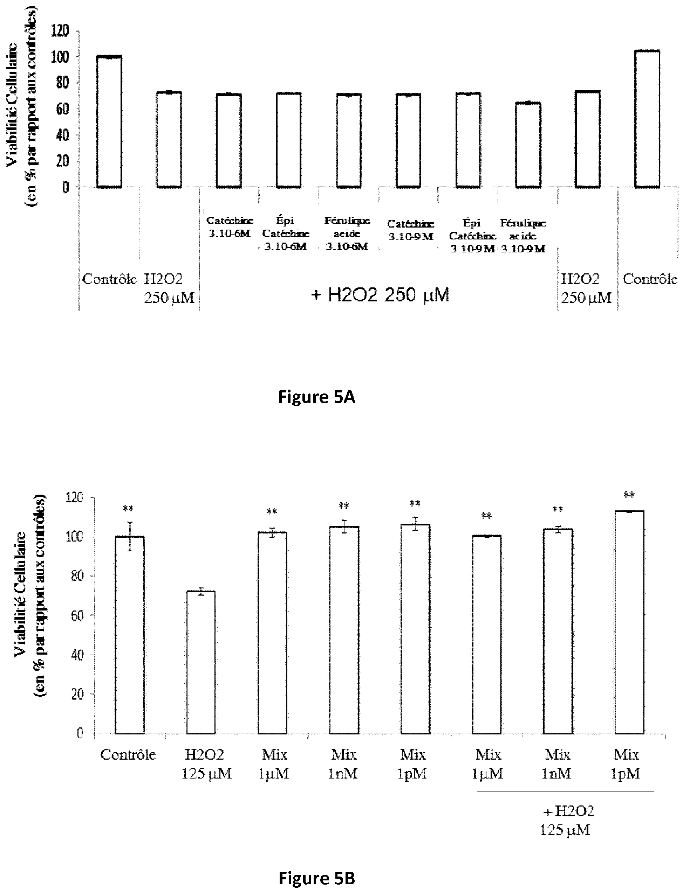

Les cellules neuronales SK-N-SH ont été soumises à une concentration toxique de peroxyde d'hydrogène (250 µM) et co-traitées avec de l'epicatéchine, de la catéchine ou de l'acide férulique à 1 µM, 1nM et 1pM pendant 24 heures. A la fin du traitement, les cellules ont été lavées deux fois et la mort cellulaire (libération de LDH) et la survie (test Résazurine) ont été analysées. Les résultats présentant les effets des trois polyphénols pris individuellement après ce traitement aigu sont présentés sur la Figure 5A. On constate que les polyphénols pris individuellement ne protègent pas les cellules contre le peroxyde d'hydrogène.

De façon à étudier l'effet synergique de l'épicatéchine, de la catéchine et de l'acide férulique, les cellules neuronales SK-N-SH ont été soumises pendant 24 heures à une concentration toxique en peroxyde d'hydrogène et co-traitées avec un mélange (Mix) comprenant à la fois de l'épicatéchine, de la catéchine et de l'acide férulique, ledit mélange étant testé aux différentes concentrations de 1µM, 1nM ou 1pM. A la fin du traitement, les cellules ont été lavées deux fois et la mort cellulaire (libération de LDH) et la survie (test Résazurine) ont été analysés. Les résultats présentant les effets des trois polyphénols ensemble après ce traitement aigu sont présentés sur les figures 5B. On constate que la combinaison des trois polyphénols ne protège pas les cellules contre le peroxyde d'hydrogène.

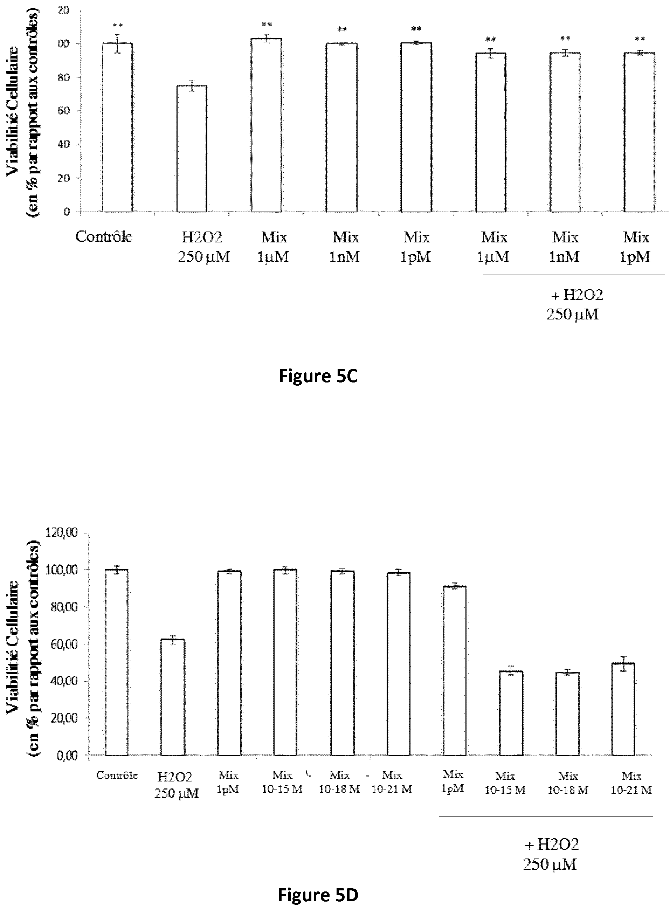

Les effets neuroprotecteurs des trois polyphénols, i.e. épicatéchine, catéchine et acide férulique, avec un traitement cumulatif ont ensuite été étudiés. Les cellules SK-N-SH ont été cultivées jusqu'à 80% de confluence, puis ensemencées dans des plaques multi-puits de culture cellulaire. Le jour suivant, chacun des trois polyphénols séparément a été ajouté au milieu à 1µM, 1nM et 1pM pendant 3 jours consécutifs. Au troisième jour, les cellules sont soumises à une concentration toxique de peroxyde d'hydrogène (250 µM) et la protection a été analysée 24 heures après. A la fin du traitement, les cellules ont été lavées deux fois et la survie cellulaire (test de Résazurine) a été analysée. Les résultats présentant les effets des trois polyphénols pris individuellement (Mix) après ce traitement cumulatif sont présentés sur la Figure 5C. On constate que les polyphénols pris individuellement ne protègent pas les cellules contre le peroxyde d'hydrogène après 3 jours de traitement consécutif.

Afin d'étudier l'effet synergique de l'épicatéchine, de la catéchine et de l'acide férulique, les cellules SK-N-SH ont été cultivées jusqu'à 80% de confluence, puis ensemencées dans des plaques multi-puits de culture cellulaire. Le jour suivant, un mélange des trois polyphénols a été ajouté au milieu pendant 3 jours consécutifs, chaque polyphénol étant présent à une concentration de 1µM, 1nM ou 1pM dans le mélange. Au troisième jour, les cellules ont été soumises à une concentration toxique de peroxyde d'hydrogène (125µM et 250 µM) et la protection a été analysée 24 heures après. A la fin du traitement, les cellules ont été lavées deux fois et la survie cellulaire (test de Résazurine) a été analysée. Les résultats présentant les effets du mélange des trois polyphénols (Mix) après ce traitement cumulatif sont présentés sur la Figure 5D. On constate que le mélange de l'épicatéchine, de la catéchine et de l'acide férulique à 1 µM or 1 nM or 1 pM protège les cellules contre le peroxyde d'hydrogène après 3 jours de traitement consécutif.

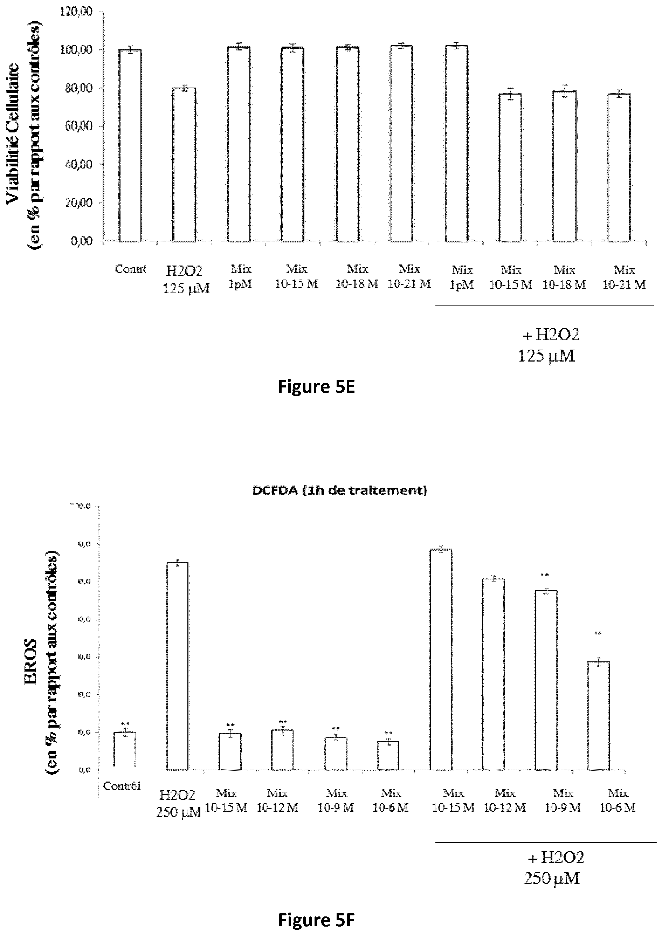

Le même essai a été réalisé avec des concentrations plus faibles dans le mélange des trois polyphénols : 10⁻¹⁵ M, 10⁻¹⁸ M ou 10⁻²¹ M de chaque polyphénol dans le mélange (Mix). On constate sur la Figure 5E qu'avec ces concentrations plus faibles, le mélange ne protège pas les cellules contre des concentrations toxiques en peroxyde d'hydrogène. Ceci montre bien que la quantité des trois polyphénols spécifiques dans l'agent selon l'invention est important pour obtenir l'effet synergique recherché.

Comme le mélange des trois polyphénols en une quantité suffisante est capable de protéger les cellules neuronales SK-N-SH après un traitement cumulatif, une étude de l'effet du mélange sur la production intracellulaire de ROS (espèces réactives de l'oxygène) a ensuite été réalisée. Pour cela, les cellules ont été cultivées jusqu'à 80% de confluence, puis ensemencées dans des plaques multi-puits de culture cellulaire. Le jour suivant, un mélange contenant les trois polyphénols, i.e. de l'épicatéchine, de la catéchine et de l'acide férulique a été ajouté au milieu, chaque polyphénol étant présent à une concentration de 1µM, 1nM ou 1pM dans le mélange. Au troisième jour, les cellules SK-N-SH ont été soumises à une concentration toxique de peroxyde d'hydrogène (250 µM) et le niveau de ROS dans les cellules a été mesuré en utilisant une sonde fluorescente DCFDA. Les résultats obtenus présentés sur la figure 5F montrent que le mélange des trois polyphénols (Mix) contenant1µlM, 1nM ou 1pM de chacun des polyphénols, permet de diminuer le niveau de ROS en présence de 250 µM de peroxyde d'hydrogène.

### Essai 4 : Evaluation de l'effet synergique chez le chien de l'agent selon l'invention

L'objectif de cet essai est de vérifier l'efficacité d'un agent selon l'invention (mélange de l'exemple 2) sur le statut antioxydant de chiens adultes en comparant cette efficacité à celle d'un extrait de Vitis vinifera, d'un extrait de *Vaccinium angustifolium* et d'un contrôle.

Neuf chiens Beagle (6 males et 3 femelles, BCS (« body condition score » état corporel) 5/9, âge moyen 20± 0.9 mois, poids moyen 9,1 ± 0.4 kg) ont été nourris avec un régime d'entretien pour maintenir leur poids corporel. L'alimentation des chiens a été supplémentée avec des capsules de gélatine (Cooper, Melun Cedex, France) contenant soit :
- une maltodextrine (Placebo contrôle; Glucidex12 lot#421A323532, Roquette, Lestrem Cedex, France),
- un extrait de Vitis vinifera (Grape; Neurogrape Inside PC PR120 batchAl50288, Activ'Inside, Libourne, France),
- un extrait de *Vaccinium angustifolium* (Wild blueberry extract 0.4TP lot#294, Nutra Canada, Québec, Canada),
- le mélange de l'exemple 2 (4 mg/kg de poids corporel /jour).

L'expérience a été conçue selon un test croisé où les chiens ont été nourris avec des rations expérimentales avec la capsule de supplémentation pendant 28 jours avec une semaine de pause entre chaque période de supplémentation. Chaque chien a ainsi reçu chacune des quatre supplémentations.

Des échantillons de sang ont été prélevés dans la veine jugulaire avant et après chaque supplémentation, et ont été maintenus dans de la glace. Le plasma a été récupéré par centrifugation à 2124g du sang total pendant 10 min à 4 ° C. Des aliquotes de plasma ont été incubés à 80 ° C.

Le statut oxydant a été évalué en mesurant le statut antioxydant total (TAS « Total Antioxydant Status »). Pour cela, un test colorimétrique des laboratoires RANDOX (Ref: NX2332, Crumlin, County Antrim, UK) a été utilisé pour évaluer le TAS. La méthode consiste à incuber du 2,2'-Azino-di-[3-ethylbenzthiazoline sulphonate] (ABTS) avec une peroxydase (métmyoglobine) et du peroxyde d'hydrogène pour produire le cation radicalaire ABTS⁺. Il présente une couleur bleu-vert relativement stable, mesurée à 600nm. La présence d'antioxydants dans les échantillons entraîne la suppression de la production de cette couleur à un degré proportionnel à leur concentration. Le TAS est exprimé en mmol/L.

Pour comparer les quatre régimes les uns avec les autres, le ΔTAS a été déterminé en comparant le TAS avant et après supplémentation : ΔTAS = TAS jour 28 - TAS Baseline.

Le ΔTAS a été analysé en utilisant un modèle mixte. Ce modèle inclut les effets fixes de la Baseline, le traitement et l'ordre de randomisation. Il a été mis en œuvre avec SAS (v9.4), procédure mixte avec une corrélation matrice non structurée pour modéliser les erreurs dans les animaux. Les paramètres ont été estimés en utilisant la méthode du maximum de vraisemblance restreint avec l'algorithme de Newton-Raphson. Le dénominateur de degrés de liberté a été estimé en utilisant l'approximation de Satterthwaite. Tous les effets ont été évalués avec un degré α = 0,10.

Le test de Wilcoxon a été utilisé pour comparer les changements de valeur de TAS avant et après supplémentation.

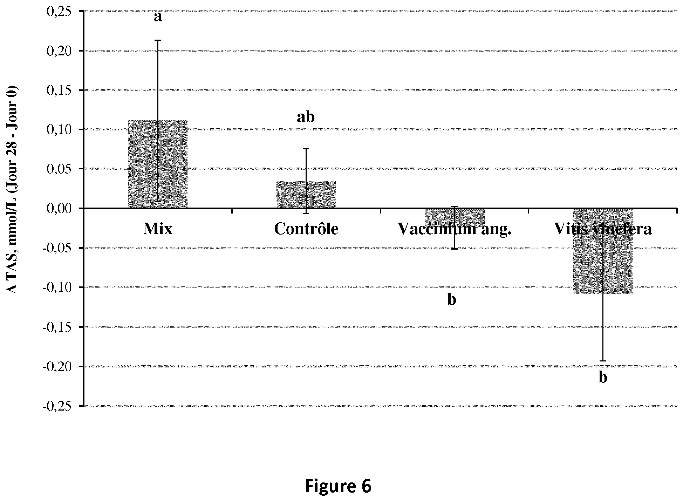

Les résultats obtenus sont présentés dans le tableau 4 (moyenne, erreur type et test de Wilcoxon) et sur la Figure 6.

**Tableau 4 : TAS (mmol/L) (Moyenne ± SEM) pour des groupes de chiens adultes (n = 9) nourris avec différents régimes de supplémentation : agent selon l'invention (Invention), extrait de Vitis vinifera (Vv), extrait de Vaccinium angustofolium (Va) ou maltodextrine (Contrôle) pendant 28 jours**

| | | **Jour 0** | | **Jour 28** | | **p-value (Wilcoxon)** |
|---|---|---|---|---|---|---|
| | | Moyenne | Erreur-type | Moyenne | Erreur-type | Traitement |
| **TAS mmol/L** | **Va** | 0,92 | 0,122 | 0,90 | 0,109 | NS |
| | **Invention** | 0,81 | 0,052 | 0,93 | 0,070 | 0,023 |
| | **Vv** | 0,97 | 0,155 | 0,87 | 0,077 | NS |
| | **Contrôle** | 0,84 | 0,048 | 0,88 | 0,075 | NS |

On constate dans le tableau et sur la Figure 6, que le mélange selon l'invention augmente significativement et de manière synergique la concentration TAS en comparaison à l'extrait de *Vitis vinifera* ou l'extrait de *Vaccinium angustofolium* seuls.

De plus, les résultats du test de Wilcoxon montrent que l'invention est la seule supplémentation qui présente des concentrations TAS significativement élevées après supplémentation. La supplémentation avec des extraits de *Vitis vinifera* ou l'extrait de *Vaccinium angustofolium* seuls ne modifie pas signification la concentration TAS.

Ainsi, la supplémentation avec un mélange de molécules provenant de *Vitis vinifera* et de *Vaccinium angustofolium* selon l'invention, a un effet synergique sur le statut antioxidant total des animaux en comparaison aux supplémentations avec un extrait de *Vitis vinifera* et un extrait de *Vaccinium angustofolium* seuls.

### Essai 5 : Evaluation de l'effet sur la mémoire

L'objectif de cette étude est de vérifier l'effet d'un mélange selon l'invention (exemple 2) à deux doses sur les niveaux de mémoire chez les chiens.

L'étude est une étude préclinique randomisée en aveugle, dans laquelle un modèle de groupe parallèle longitudinal a été utilisé. Trente-cinq chiens Beagle (Vivocore Inc. colony, Toronto, Canada; 14 mâles et 21 femelles; âgés entre 8.0 to 14.5 ans au début de l'étude) ont été répartis en trois groupes pour l'expérience trois semaines avant le début des supplémentations. La répartition des chiens a été faite en fonction des performances (scores cumulatifs) sur la base des scores au test DNMP (« delayed non-matching position »), afin que chaque groupe ait un score total sensiblement équivalent au test DNMP.

Les trois groupes de chiens ont ensuite été respectivement nourris avec des croquettes contenant en inclusion soit 0 ppm (placebo), soit 240 ppm du mélange de l'exemple 2, soit 480 ppm du mélange de l'exemple 2 (ppm par rapport au poids de croquette).

Le test DNMP a été réalisé des jours - 27 à -16, et l'analyse a été réalisée des jours 58 à 63. Le test DNMP test comprend deux phases :
- Phase 1 : le chien doit déplacer un objet placé sur l'une des trois positions possibles sur un puits de nourriture. Le bloc à déplacer couvre une récompense.
- Phase 2 : après une attente de 20s à 90s, deux objets identiques à la première phase sont présentés au chien. Un objet est localisé à la même position que lors de la première phase. Néanmoins le bon objet est placé à l'une des deux positions restantes (pas de correspondance), et si le chien déplace cet objet, il reçoit la récompense.

Pour la présente étude, 12 sessions de test DNPM ont été réalisées, avec une cession de test par jour pour tous les sujets. La sous-tâche à délai variable a été utilisée. Pour chaque cession de test, des délais de 20 et 90 secondes ont été répartis de façon équitable dans les 12 essais, permettant l'évaluation de la mémoire de travail. Un intervalle inter- essai de 30 secondes a été appliqué. Il y a eu 6 sessions pour la phase initiale et six sessions pour la phase de traitement. Les sujets ont été testés sur chacun des jours désignés indépendamment du score. Au cours de toutes les procédures de test, les animaux ont été récompensés avec de la nourriture humide pour chien en conserve Purina Essential Care Adulte Formule.

Afin de comparer les trois traitements sur les résultats DNMP, l'augmentation en regard de la Baseline a été analysée en utilisant un test Chi-squared. L'analyse a été réalisée en utilisant SAS (v9.4) avec un degré α = 0.05.

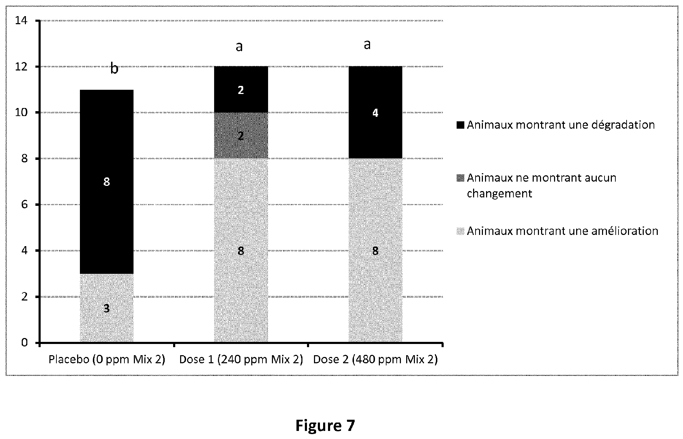

Les résultats sont présentés sur la Figure 7. Une augmentation cognitive significative est observée lorsque les chiens ont reçu un traitement selon l'invention (Mix2) en comparaison aux chiens n'ayant pas reçu le traitement. De plus, la quantité de traitement n'affecte pas le nombre de chiens présentant une augmentation significative, ce qui montre que, chez le chien, l'efficacité est indépendante de la dose de traitement.

### Essai 6 : Evaluation de l'effet sur la tolérance du chien

Le but de cet essai était de vérifier la sécurité alimentaire de l'agent nutritionnel ou thérapeutique de l'invention sur le chien, en vérifiant certains biomarqueurs rénaux. En effet, de nombreuses publications ont fait état d'une toxicité du raisin sur le chien, provoquant chez celui-ci des déficiences rénales dont les symptomes se traduisent par des vomissements, diahrrées, etc. (Eubig P, Brady M, Gwaltney-Brant S, Khan S, Mazzaferro E, Morrow C (2005). "Acute renal failure in dogs after the ingestion of grapes or raisins: a retrospective evaluation of 43 dogs" (1992-2002).

Vingt- quatre chiens Beagle (20 mâles et 4 femelles, BCS 5/9, moyenne d'âge 31± 3 mois, poids moyen 11.4 ± 0.2 kg), ont été nourris avec un régime alimentaire de maintien (Royal Canin medium adult, France) afin de maintenir leur poids optimal durant l'essai. Quatre groupes de 6 chiens ont ainsi chacun reçu des suppléments, i.e. des capsules contenant de la matlodextrine (placebo) ou un agent selon l'invention, i.e. le mix tel que préparé à l'exemple 2 à : 4 (Mix 1), 20 (Mix 5), 40 (Mix 10) mg/kg de poids corporel/jour.

Des échantillons d'urine et de sang ont été prélevés au début de l'essai (semaine 0), après 12 semaines et après 24 semaines.

La cystatine C (CysC), clustérine et neutrophil gelatinase-associated lipocalin (NGAL) du plasma et de l'urine ont été analysés dans les échantillons de sang (Tvarijonaviciute A, Ceron JJ, Holden SL, Biourge V, Morris PJ, German AJ. Effect of Weight Loss in Obese Dogs on Indicators of Renal Function or Disease. J. Vet. Intern. Med. 2013;27:31-38.

Garcia-Martinez JD, Tvarijonaviciute A, Ceron JJ, Calden M, martinez-Subierla S. Urinary Clusterin as a Renal Marker in Dogs. J. Vet. Diagn. Invest. 2012;24:301-306). Chaque paramètre a été analysé au moyen d'un modèle à effet mixte, en évaluant les effets de la ligne de base, jour, traitement ainsi que jour x de traitement (SAS v9.4 ; α = 0.05)..

Comme montré sur la tableau 5, les biomarqueurs ont été trouvés en quantités inférieures à la limite supérieure obtenue avec le contrôle et les traitements expérimentaux à semaine 0 (plasma CysC, CysC urinaire/Crea ratio, clusterin urinaire/Creat ratio, plasma NGAL, NGAL urinaire/Creat respectivement de 2.23 µg/mL, 156 ng/g, 443 ng/g, 47 ng/mL, 28.5 ng/g ).

**Tableau 5**

| | | **CysC, µg/mL** | | **CysC urinaire/Creat ratio, µg/g** | | **Clusterin urinaire /Creat ratio, ng/g** | | **NGAL, ng/mL** | | **NGAL urinaire /Creat ratio, ng/g** | |
|---|---|---|---|---|---|---|---|---|---|---|---|
| | | Mean | SEM | Mean | SEM | Mean | SEM | Mean | SEM | Mean | SEM |
| **Semaine 0** | **Contrôle** | 1,25 | 0,085 | 18,9 0 | 17,24 3 | 79,3 | 20,0 5 | 17,8 | 4,52 | 11,1 2 | 4,68 8 |
| | **Mix 1** | 1,25 | 0,15 2 | 30,3 8 | 25,39 0 | 44,5 | 13,2 2 | 17,0 | 5,02 | 7,97 | 4,48 7 |
| | **Mix 5** | 1,17 | 0,08 4 | 27,3 7 | 14,26 1 | 63,4 | 27,5 3 | 14,9 | 3,49 | 4,18 | 1,78 6 |
| | **Mix 10** | 1,47 | 0,16 1 | 12,2 0 | 4,441 | 83,7 | 22,6 7 | 18,0 | 3,00 | 5,45 | 1,01 9 |
| **Semaine 12** | **Contrôle** | 1,18 | 0,07 5 | 8,25 | 3,156 | 61,1 | 34,0 7 | 23,8 | 5,21 | 8,42 | 3,69 0 |
| | **Mix 1** | 1,27 | 0,17 3 | 9,83 | 4,594 | 67,9 | 33,3 3 | 15,3 | 4,69 | 5,68 | 1,93 4 |
| | **Mix 5** | 1,22 | 0,07 9 | 5,12 | 1,696 | 39,5 | 17,1 6 | 12,9 | 2,80 | 4,00 | 1,55 1 |
| | **Mix 10** | 1,35 | 0,09 9 | 12,9 3 | 6,086 | 98,7 | 52,5 9 | 19,7 | 4,03 | 4,72 | 1,55 2 |
| **Semaine 24** | **Contrôle** | 1,10 | 0,13 2 | 14,2 7 | 9,840 | 135, 1 | 66,1 0 | 23,7 | 2,67 | 7,33 | 2,48 3 |
| | **Mix 1** | 1,23 | 0,18 2 | 4,13 | 1,406 | 94,2 | 69,2 6 | 19,3 | 4,80 | 4,92 | 1,35 2 |
| | **Mix 5** | 1,18 | 0,07 9 | 21,4 2 | 11,94 1 | 56,2 | 24,7 2 | 14,3 | 4,04 | 3,52 | 0,29 7 |
| | **Mix 10** | 1,30 | 0,11 6 | 3,80 | 1,240 | 47,5 | 20,2 3 | 20,7 | 3,53 | 4,55 | 1,39 3 |
| **p-values** | **traitement** | NS | | NS | | NS | | NS | | NS | |
| | **jour** | NS | | NS | | NS | | NS | | NS | |
| | **Traitement x jour** | NS | | NS | | NS | | NS | | NS | |

Les chiens adultes consommant l'agent selon l'invention ne montrent donc aucun signe clinique d'intolérance, et ce même à la dose maximum testée (10 fois la dose normale).

### Essai 7 : Effet sur la maladie d'Alzheimer

Cet essai a été réalisé sur un modèle de souris hétérozygote 3xTg-AD telles que décrites par exemple dans Arsenault D, Dal-Pan A, Tremblay C, Benett DA, Guitton MJ, et al. (2013) PAK Inactivation Impairs Social Recognition in 3xTg-AD Mice without increasing Barin Deposition of Tau and Aβ. The journal of Neuroscience 33: 10729-10740.

120 souris non transgéniques (Non Tg) et triple transgéniques (3xTg-AD) agées de 12 mois ont été utilisées pour cet essai. Sept souris sont mortes avant l'essai et ont été exclues.

En complément, un groupe additionnel de souris de 4 mois C57BL/6 a été utilisé comme gourpe contrôle.

L'agent selon l'invention de l'exemple 1 a été introduit dans des pellets pour souris. Les souris ont été nourries pendant 4 mois avec une alimentation contrôle ou avec 500 mg d'extrait/kg de poids corporel /jour (référence "Polyph1") ou 2500 mg d'extraits/kg de poids corporel/jour (référence "Polyph2").

Trois mois après le début de l'essai, des analyses comportementales ont été réalisées. Après un mois supplémentaires, les souris sont placées sous anesthésie profonde et des extraits de sang intracardiaque ont prélevés. Elles sont ensuite perfusées avec une perfusion intracardiaque avec un tampon phosphate salin contenant des inhbiteurs de protéases et des inhibiteurs de phosphatases. Des extraits de cortex parieto-temporal ont ensuite été disséqués, congelés et maintenus à -80°C. Ils sont ensuite traités pour être analysés par elisa, western blott et immunofluorescence pour mesurer les marqueurs suivants : beta-amyloide (Aβ, protéine Tau et facteur neutrophique issu du cerveau BDNF (« Barin derived neurotrophic factor).

Tout d'abord, on constate un déclin cognitif chez les souris de 15 mois 3xTg-AD et nonTg en comparaison à des souris de 4 mois non-Tg (contrôle). On constate en effet pour les souris âgées une diminution du ratio de reconnaissance ou index de reconnaissance avec le test de reconnaissance d'un nouvel objet. De façon avantageuse, l'administration de l'agent selon l'invention à des doses de 500 ou 2500 mg/kg/jour prévient la détérioration de la mémoire chez les souris 3xTg-AD.

Par ailleurs l'agent selon l'invention permet de prévenir la diminution de BDNF (facteur neurotrophique issu du cerveau) chez les souris de 16 mois 3xTg-AD.

Enfin, les résultats obtenus montrent que les concentrations en métabolites phénoliques sont corrélées avec les performance cognitives (mémoire) des souris supplémentées avec l'agent selon l'invention.

## Revendications

1. Agent nutritionnel ou thérapeutique comprenant au moins un mélange de molécules obtenu à partir d'un extrait de *Vitis vinifera* et d'un extrait de *Vaccinium angustifolium,* comprenant :
- au moins 1% de catéchines et/ou épicatéchines, le pourcentage étant donné en poids par rapport au poids total du mélange,
- au moins 5 ppm (parties par million dans le mélange) d'acide férulique, et
- au moins 200ppm de resvératrol,
- au moins 300ppm de la malvidine 3 glucoside,
- au moins 50ppm de quercétine et/ou glycosides de quercétine, et
- au moins 500ppm d'anthocyanidines.

2. Agent selon la revendication 1, **caractérisé en ce que** le mélange de molécules comprend au moins 5% de catéchines et/ou épicatéchines, le pourcentage étant donné en poids par rapport au poids total du mélange.

3. Agent selon la revendication 1 ou 2, **caractérisé en ce que** le mélange de molécules comprend au moins 10 ppm (parties par million dans le mélange) d'acide férulique.

4. Agent selon la précédente revendication, **caractérisé en ce que** l'extrait de *Vitis vinifera* présente une teneur en polymères de flavanols inférieure à 0,5% en poids total des polyphénols de l'extrait.

5. Agent selon l'une des précédentes revendications, pour une utilisation comme médicament pour l'Homme ou l'animal.

6. Agent pour une utilisation selon la revendication 5, à une dose permettant de fournir à l'Homme ou l'animal :
- au moins 100 µg par kg de poids corporel de catéchines et/ou d'épicatéchines,
- au moins 0,05 µg par kg de poids corporel d'acide férulique, et
- au moins 10 µg par kg de poids corporel de resvératrol.

7. Agent pour une utilisation selon l'une des revendications 5 ou 6, à une dose permettant de fournir à l'Homme ou l'animal :
- au moins 0,2 µg par kg de poids corporel de quercétine et/ou glycosides quercétines, et
- au moins 1 µg par kg de poids corporel d'anthocyanidines.

8. Utilisation non thérapeutique d'un agent selon l'une des revendications 1 à 4 chez l'Homme ou l'animal sain, pour améliorer les fonctions cognitives et/ou les fonctions exécutives, et/ou pour limiter le déclin cognitif non pathologique lié à l'âge.

9. Utilisation non thérapeutique selon la revendication 8, **caractérisée en ce que** ledit agent est utilisé à une quantité permettant de fournir à l'Homme ou l'animal :
- au moins 100 µg par kg de poids corporel de catéchines et/ou d'épicatéchines,
- au moins 0,05 µg par kg de poids corporel d'acide férulique, et
- au moins 10 µg par kg de poids corporel de resvératrol.

10. Utilisation non thérapeutique selon l'une des revendications 8 ou 9, **caractérisée en ce que** ledit agent est utilisé à une quantité permettant de fournir à l'Homme ou l'animal :
- au moins 0,2 µg par kg de poids corporel de quercétine et/ou glycosides quercétines, et
- au moins 1 µg par kg de poids corporel d'anthocyanidines.

11. Composition comprenant un agent nutritionnel ou thérapeutique selon l'une des revendications 1 à 4, **caractérisée en ce qu'**elle se présente sous une forme choisie parmi les comprimés, les capsules, les gélules, les poudres, les solutions, les microcapsules, les suspensions, les émulsions, les compléments alimentaires, les boissons, et les aliments pour l'Homme ou l'animal.

## Patentansprüche

1. Ernährungs- oder therapeutisches Mittel, umfassend mindestens eine Mischung von Molekülen, die aus einem Extrakt von *Vitis vinifera* und einem Extrakt von *Vaccinium angustifolium* erhalten wird, umfassend:
- mindestens 1 % Catechine und/oder Epicatechine, wobei der Prozentsatz als Gewicht bezogen auf das Gesamtgewicht der Mischung gegeben ist,
- mindestens 5 ppm (Teile pro Million in der Mischung) Ferulasäure, und
- mindestens 200 ppm Resveratrol,
- mindestens 300 ppm Malvidin-3-glucosid,
- mindestens 50 ppm Quercetin und/oder Glycoside von Quercetin, und
- mindestens 500 ppm Anthocyanidine.

2. Mittel nach Anspruch 1, **dadurch gekennzeichnet, dass** die Mischung von Molekülen mindestens 5 % Catechine und/oder Epicatechine umfasst, wobei der Prozentsatz als Gewicht bezogen auf das Gesamtgewicht der Mischung gegeben ist.

3. Mittel nach Anspruch 1 oder 2, **dadurch gekennzeichnet, dass** die Molekülmischung mindestens 10 ppm (Teile pro Million in der Mischung) Ferulasäure umfasst.

4. Mittel nach dem vorstehenden Anspruch, **dadurch gekennzeichnet, dass** der Extrakt aus *Vitis vinifera* einen Gehalt an Flavanolpolymeren von weniger als 0,5 % des Gesamtgewichts von Polyphenolen des Extrakts aufweist.

5. Mittel nach einem der vorstehenden Ansprüche für eine Verwendung als Arzneimittel für den Menschen oder das Tier.

6. Mittel zur Verwendung nach Anspruch 5 in einer Dosis, die es ermöglicht, dem Menschen oder dem Tier bereitzustellen:
- mindestens 100 µg pro kg Körpergewicht Catechine und/oder Epicatechine,
- mindestens 0,05 µg pro kg Körpergewicht Ferulasäure, und
- mindestens 10 µg pro kg Körpergewicht Resveratrol.

7. Mittel zur Verwendung nach einem der Ansprüche 5 oder 6 in einer Dosis, die es ermöglicht, dem Menschen oder dem Tier bereitzustellen:
- mindestens 0,2 µg pro kg Körpergewicht Quercetin und/oder Quercetinglycoside, und
- mindestens 1 µg pro kg Körpergewicht Anthocyanidine.

8. Nicht therapeutische Verwendung eines Mittels nach einem der Ansprüche 1 bis 4 bei dem gesunden Menschen oder Tier zum Verbessern der kognitiven Funktionen und/oder der exekutiven Funktionen und/oder zum Begrenzen des altersbedingten, nicht pathologischen kognitiven Abbaus.

9. Nicht therapeutische Verwendung nach Anspruch 8,
**dadurch gekennzeichnet, dass** das Mittel in einer Menge verwendet wird, die es ermöglicht, dem Menschen oder dem Tier bereitzustellen:
- mindestens 100 µg pro kg Körpergewicht Catechine und/oder Epicatechine,
- mindestens 0,05 µg pro kg Körpergewicht Ferulasäure, und
- mindestens 10 µg pro kg Körpergewicht Resveratrol.

10. Nicht therapeutische Verwendung nach einem der Ansprüche 8 oder 9, **dadurch gekennzeichnet, dass** das Mittel in einer Menge verwendet wird, die es ermöglicht, dem Menschen oder dem Tier bereitzustellen:
- mindestens 0,2 µg pro kg Körpergewicht Quercetin und/oder Quercetinglycoside, und
- mindestens 1 µg pro kg Körpergewicht Anthocyanidine.

11. Zusammensetzung, umfassend ein Nährstoff- oder therapeutisches Mittel nach einem der Ansprüche 1 bis 4, **dadurch gekennzeichnet, dass** sie in einer Form vorliegt, die aus Tabletten, Kapseln, Hartkapseln, Pulvern, Lösungen, Mikrokapseln, Suspensionen, Emulsionen, Nahrungsergänzungsmitteln, Getränken und Lebensmitteln für Mensch oder Tier ausgewählt ist.

## Claims

1. Nutritional or therapeutic agent comprising at least a mixture of molecules obtained from an extract of *Vitis vinifera* and from an extract of *Vaccinium angustifolium*, the mixture comprising:
- at least 1% of catechins and/or epicatechins, the percentage being given by weight relative to the total weight of the mixture,
- at least 5 ppm (parts per million in the mixture) of ferulic acid, and
- at least 200 ppm of resveratrol,
- at least 300 ppm of malvidin-3-glucoside,
- at least 50 ppm of quercetin and/or quercetin glycosides, and
- at least 500 ppm of anthocyanidins.

2. Agent according to claim 1, **characterized in that** the mixture of molecules comprises at least 5% of catechins and/or epicatechins, the percentage being given by weight relative to the total weight of the mixture.

3. Agent according to claim 1 or 2, **characterized in that** the mixture of molecules comprises at least 10 ppm (parts per million in the mixture) of ferulic acid.

4. Agent according to the preceding claim, **characterized in that** the *Vitis vinifera* extract has a flavanol polymer content of less than 0.5% by total weight of the polyphenols of the extract.

5. Agent according to any of the preceding claims, for use as a drug for human beings or animals.

6. Agent for use according to claim 5, at a dose providing human beings or animals with:
- at least 100 µg per kg of body weight of catechins and/or epicatechins,
- at least 0.05 µg per kg of body weight of ferulic acid, and
- at least 10 µg per kg of body weight of resveratrol.

7. Agent for use according to either of claims 5 or 6, at a dose providing human beings or animals with:
- at least 0.2 µg per kg of body weight of quercetin and/or quercetin glycosides, and
- at least 1 µg per kg of body weight of anthocyanidins.

8. Non-therapeutic use of an agent according to any of claims 1 to 4 in healthy human beings or animals in order to improve cognitive functions and/or executive functions, and/or to limit non-pathological age-related cognitive decline.

9. Non-therapeutic use according to claim 8, **characterized in that** said agent is used at an amount providing human beings or animals with:
- at least 100 µg per kg of body weight of catechins and/or epicatechins,
- at least 0.05 µg per kg of body weight of ferulic acid, and
- at least 10 µg per kg of body weight of resveratrol.

10. Non-therapeutic use according to either of claims 8 or 9,
**characterized in that** said agent is used at an amount providing human beings or animals with:
- at least 0.2 µg per kg of body weight of quercetin and/or quercetin glycosides, and
- at least 1 µg per kg of body weight of anthocyanidins.

11. Composition comprising a nutritional or therapeutic agent according to any of claims 1 to 4, **characterized in that** the composition is in a form selected from tablets, capsules, gel capsules, powders, solutions, microcapsules, suspensions, emulsions, food supplements, beverages, and foods for human beings or animals.
